# EUROPEAN PATENT APPLICATION

(11) **EP 4 459 281 A1**
(43) Date of publication of application: **06.11.2024**
(21) Application number: 22916103.9
(22) Date of filing: 27.12.2022
(51) Int. Cl.: G01N 33/53, G01N 33/543

(54) **DETECTION METHOD AND DETECTION REAGENT**

(30) Priority: 28.12.2021 JP 2021214780
(71) Applicant: Sekisui Medical Co., Ltd., Tokyo 103-0027 (JP)
(72) Inventor: YAMADA Daisuke, Tokyo 103-0027 (JP); SUZUKI Ikumi, Tokyo 103-0027 (JP); FUJIMURA Kengo, Tokyo 103-0027 (JP); ASAI Tomohide, Tokyo 103-0027 (JP); MIYAZAKI Osamu, Tokyo 103-0027 (JP)
(74) Representative: Ter Meer Steinmeister & Partner
(86) International application number: PCT/JP2022/048208
(87) International publication number: WO 2023/127881

(57) **Abstract**

It is an object of the present invention to provide a method for suppressing non-specific reactions using a highly versatile method without affecting specific reactions. The present invention provides a detection method for detecting a target antigen in a sample using a specific antibody that specifically binds to the antigen, the method comprising: a) a step of bringing the sample into contact with a modified antibody; and b) a step of bringing the sample into contact with the specific antibody simultaneously with or after the step a), which is a highly versatile method that can suppress non-specific reactions.

## Description

### [Technical Field]

The present invention relates to a detection method and detection reagent using a specific antibody against an antigen in a sample. More specifically, the present invention relates to the detection method characterized in that, in order to suppress non-specific reactions, a modified antibody of a specific antibody is used, and the method comprises:
a) a step of bringing a sample into contact with the modified antibody; and
b) a step of bringing the sample into contact with the specific antibody simultaneously with or after the step a), and also relates to the detection reagent.

### [Background Art]

As a measurement method in the field of diagnostic drugs, there is an immunoassay method that uses a specific antibody to detect a measurement target antigen that is present in a biological sample.

In addition to the measurement target antigen, the biological sample contains various substances, and often contains substances called non-specific factors that cause non-specific reactions. The presence of nonspecific factors in a sample promotes binding that is not based on specific reactions or prevents specific immune reactions, leading to measurement errors. Although the presence of heterophilic antibodies and rheumatoid factor (RF) has been clarified as non-specific factors, it is presumed that there are various other substances, and many unclarities remain.

Incidentally, as techniques for suppressing non-specific reactions in immunoassays, for example, those described in Patent Literatures 1 to 4 are known. However, these methods cannot in principle be applied to all measurement items, and there are actually measurement items in which nonspecific reactions cannot be suppressed.

Patent Literature 1 describes a measurement method in which non-specific reactions are suppressed by performing an immune reaction in the copresence of a monoclonal antibody-derived substance that has completely or substantially lost the specific antibody activity inherent in the monoclonal antibody used in immunoassay, but has substantially retained its non-specific activity. Specifically, the monoclonal antibody-derived substance is a substance prepared so as to substantially eliminate the original antibody activity of the antibody by denaturing the structure of the antibody by ultrasonic treatment, organic solvent treatment, acid/alkali treatment, etc., but substantially retain non-specific activity.

Patent Literature 2 describes a method for detecting an antigen to be detected in a sample using an insoluble carrier having a fragment antibody supported thereon, in which a modified product of the fragment antibody is brought into contact with the sample in advance or simultaneously to suppress non-specific reactions. With respect to the modified product, the modification is also implemented by methods commonly used for protein denaturation, such as heating, acid, alkali, reducing agent, and chaotropic salt.

Further, Patent Literature 3 describes an immunoassay method for detecting analytes, particularly for immunologically detecting tumor markers, which reduces interference in immunoassays by adding to a sample a substance having a peptide sequence derived from the framework region of a variable domain of a detection antibody, etc. As the peptide sequences, specific sequences present in framework region 1 and framework region 3 of the antibody heavy chain are shown.

Furthermore, Patent Literature 4 describes a method for removing the influence of interfering substances by adding an inhibitor to an immunoassay in large excess, in which the inhibitor still maintains its specificity to the interfering substance, but no longer reacts with the antigen to be analyzed, or has been altered to such an extent that the reaction decreases clearly. As methods for preparing inhibitor, the following methods are listed as examples: a method that selectively removes the antigen-binding structure from the reagent antibody by degrading the antibody using an enzyme, and a method that changes the antigen-binding structure of the reagent antibody through mutation during hybridoma culture.

### [Citation List]

### [Patent Literature]

Patent Literature 1: Japanese Patent Granted Publication No. 2561134
Patent Literature 2: Japanese Patent Granted Publication No. 6431766
Patent Literature 3: Japanese Patent Granted Publication No. 4334065
Patent Literature 4: Japanese Unexamined Patent Application Publication No. Hei 1-28560

### [Summary of Invention]

### [Technical Problem]

However, in the method of Patent Literature 1, the monoclonal antibody-derived substance that functions as a non-specific reaction inhibitor is prepared by heat treatment, etc., and it is reported that Fab is substantially removed. In order to deactivate antibody reactivity using this method, high temperature or long-term heating is required, and it is extremely difficult to find optimal conditions. When the present inventors attempted to prepare a non-specific reaction inhibitor by heat-treating a monoclonal antibody using the method described in Patent Literature 1, it was confirmed that there were cases where the deactivation of reactivity of the antibody was simultaneously accompanied by precipitate generation, resulting in no observable effect of suppressing non-specific reactions. Therefore, this method has a problem of lacking in versatility.

Further, the method of Patent Literature 2 is limited to the case where a fragment antibody is used as the detection antibody. For the modified product of fragment antibody described in Patent Literature 2, it is necessary to use an antibody that recognizes a different epitope from the fragment antibody for detection, and it is necessary to produce multiple monoclonal antibodies and clarify the epitopes recognized by the respective antibodies, which is cumbersome. Furthermore, as in the case of Patent Literature 1, it is necessary to set optimal conditions for the antibody denaturation method, so that there is a problem of poor versatility.

In addition, the method of Patent Literature 3 lacks information other than using peptides having specific sequences derived from the disclosed antibody framework region 1 and framework region 3, and it is considered that the method is only effective if the cause of non-specific reactions depends on those sequences. Therefore, as in the case of Patent Literatures 1 and 2, there is a problem of poor versatility.

Further, in the method of Patent Literature 4, a method of separating the Fab portion from the Fc portion is mentioned as a specific example of a method for decomposing an antibody using an enzyme, but the region to be separated and removed is large, and the method is considered to be effective only in the case of a non-specific reaction dependent on the Fc region, and not effective in the case of a non-specific reaction dependent on the variable region. Furthermore, because the method uses mutant antibodies that are purportedly obtained by mutations that may occur during the process of culturing hybridomas, it is extremely rare to obtain mutant antibodies with the expected mutations. Therefore, there is a problem of poor versatility and reproducibility.

The present invention aims at solving the above problems, and an object of the present invention is to provide a method for effectively suppressing non-specific reactions using a method with excellent versatility.

### [Solution to Problem]

In order to solve the above problems, the inventors of the present invention investigated whether there is a method that can suppress non-specific reactions using a highly versatile method. As a result, they have come to think that non-specific reactions might be suppressed even more reliably if it is possible to allow a substance that acts as a "decoy" for specific antibodies against the target antigen to coexist in the antigen-antibody reaction system. The present inventors have succeeded in suppressing non-specific reactions by performing an antigen-antibody reaction between the specific antibody and the target antigen in the presence of a modified antibody in which part or all of the variable region of the L chain and/or H chain of the specific antibody is modified, and completed the present invention. That is, the present invention has the following configuration.
<1> A detection method for detecting a target antigen in a sample using a specific antibody that specifically binds to the antigen, the method comprising:
   a) a step of bringing the sample into contact with a modified antibody; and
   b) a step of bringing the sample into contact with the specific antibody simultaneously with or after the step a);
   wherein the modified antibody is an antibody in which part or all of variable region of an antibody light chain (hereinafter abbreviated as L chain) and/or an antibody heavy chain (hereinafter abbreviated as H chain) of the specific antibody is modified.
<2> The detection method according to <1>, wherein the modified antibody is modified at part or all of at least one complementarity determining region (hereinafter abbreviated as CDR) and/or its region related to a three-dimensional structure of the CDR out of six CDRs of the L chain and H chain of the specific antibody, and has at least one unmodified CDR which is different from said CDR.
<3> The detection method according to <1> or <2>, wherein a reactivity of the modified antibody to the target antigen is 30 % or less relative to a reactivity of the specific antibody to the target antigen.
<4> The detection method according to any one of <1> to <3>, wherein a concentration ratio of the modified antibody to the specific antibody in an antigen-antibody reaction system is 1 to 29 times.
<5> The detection method according to any one of <1> to <4>, wherein the modified antibody is an intact antibody or a fragment antibody.
<6> The detection method according to any one of <1> to <5>, wherein the specific antibody includes one or more antibodies, and the modified antibody is a modified product of at least one of the antibodies as the specific antibody.
<7> The detection method according to any one of <1> to <6>, wherein the specific antibody includes a first specific antibody and a second specific antibody, and the modified antibody includes a modified product of at least one of the first specific antibody and the second specific antibody.
<8> The detection method according to any one of <1> to <7>, wherein the specific antibody is an intact antibody or a fragment antibody containing a binding site for the target antigen.
<9> The detection method according to any one of <1> to <8>, which is a method based on a homogeneous method or a heterogeneous method.
<10> The detection method according to any one of <1> to <9>, wherein at least one of the antibodies as the specific antibody is an antibody bound to an insoluble carrier or labeled with a labeling substance.
<11> The detection method according to <10>, wherein the insoluble carrier is latex particles, a gold colloid, a magnetic particle, a well-shaped plate, or a porous membrane.
<12> The detection method according to any one of <1> to <11>, wherein the step of bringing the target antigen in the sample into contact with the specific antibody is carried out in a solution.
<13> The detection method according to <12>, further comprising a step of optically detecting a degree of aggregation of latex particles in the solution.
<14> The detection method according to <13>, wherein the step of optically detecting comprises a step of optically detecting color development of a coloring agent or amount of a labeling substance, wherein the coloring agent or the labeling substance reacts depending on an amount of antigen-antibody complex in the solution.
<15> A detection reagent for detecting a target antigen in a sample using a specific antibody that specifically binds to the antigen, the detection reagent comprising:
   (1) a specific antibody that specifically binds to the antigen; and
   (2) a modified antibody in which part or all of variable region of a L chain and/or H chain of the specific antibody is modified.
<16> The detection reagent according to <15>, wherein the modified antibody is modified at part or all of at least one CDR and/or its region related to a three-dimensional structure of the CDR out of six CDRs of the L chain and H chain of the specific antibody, and has at least one unmodified CDR which is different from said CDR.
<17> The detection reagent according to <15> or <16>, wherein a reactivity of the modified antibody to the target antigen is 30 % or less relative to a reactivity of the specific antibody to the target antigen.
<18> The detection reagent according to any one of <15> to <17>, wherein a concentration ratio of the modified antibody to the specific antibody in an antigen-antibody reaction system is 1 to 29 times.
<19> The detection reagent according to any one of <15> to <18>, wherein the modified antibody is an intact antibody or a fragment antibody.
<20> The detection reagent according to any one of <15> to <19>, wherein the specific antibody includes one or more antibodies, and the modified antibody is a modified product of at least one of the antibodies as the specific antibody.
<21> The detection reagent according to any one of <15> to <20>, wherein the specific antibody includes a first specific antibody and a second specific antibody, and the modified antibody includes a modified product of at least one of the first specific antibody and the second specific antibody.
<22> The detection reagent according to any one of <15> to <21>, wherein the specific antibody is an intact antibody or a fragment antibody containing a binding site for the target antigen.
<23> The detection reagent according to any one of <15> to <22>, which is a reagent based on a homogeneous method or a heterogeneous method.
<24> The detection reagent according to any one of <15> to <23>, wherein at least one of the antibodies as the specific antibody is an antibody bound to an insoluble carrier or labeled with a labeling substance.
<25> The detection reagent according to <24>, wherein the insoluble carrier is latex particles, a gold colloid, a magnetic particle, a well-shaped plate, or a porous membrane.
<26> A detection reagent kit for detecting a target antigen in a sample using a specific antibody that specifically binds to the antigen, the kit comprising reagents (1) and (2), wherein at least one of the reagents (1) and (2) comprise a modified antibody in which part or all of variable region of a L chain and/or H chain of the specific antibody is modified:
   (1) a reagent containing a buffer solution, and
   (2) a reagent containing the specific antibody.
<27> A suppression method for suppressing non-specific reactions in a detection method for detecting a target antigen, the suppression method comprising using a combination of a specific antibody that specifically binds to a target antigen in a sample and a modified antibody in which part or all of variable region of a L chain and/or H chain of the specific antibody is modified.
<28> A non-specific reaction inhibitor used together with a specific antibody that specifically binds to a target antigen in a sample, comprising an antibody in which part or all of variable region of a L chain and/or H chain of the specific antibody is modified.
<29> An antibody used together with a specific antibody that specifically binds to a target antigen in a sample, which is an antibody in which part or all of variable region of a L chain and/or H chain of the specific antibody is modified.

### [Advantageous Effects of Invention]

According to the present invention, in a method for detecting a target antigen in a sample using a specific antibody that specifically binds to the antigen, an antigen-antibody reaction is performed in the presence of a modified antibody of the specific antibody, which makes it possible to broadly prevent non-specific reactions caused by non-specific factors contained in the sample and accurately measure target antigens.

### [Brief Description of Drawings]

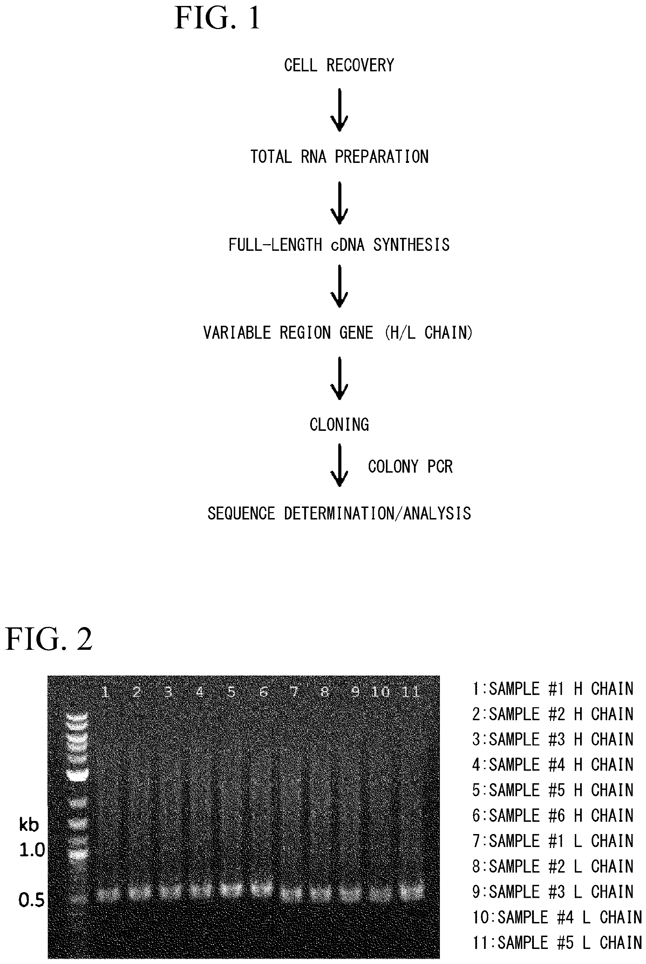

FIG.1 shows an overview of the acquisition of variable region genes from antibody-producing cells, and sequence analysis thereof. After preparing total RNA from antibody-producing cells, full-length cDNA was synthesized by reverse transcription and template switching, and H chain and L chain variable region gene fragments were acquired by PCR using primers specific for the 5' end and constant region. After cloning these, the variable region gene sequences were determined by sequencing the colony PCR amplification products.
FIG. 2 shows examples of analysis results of representative H chain and L chain variable region gene amplification fragments. A PCR amplification product of approximately 0.5 kb was detected by agarose electrophoresis. Quick-Load Purple 1kb Plus DNA Ladder was used as a size marker.

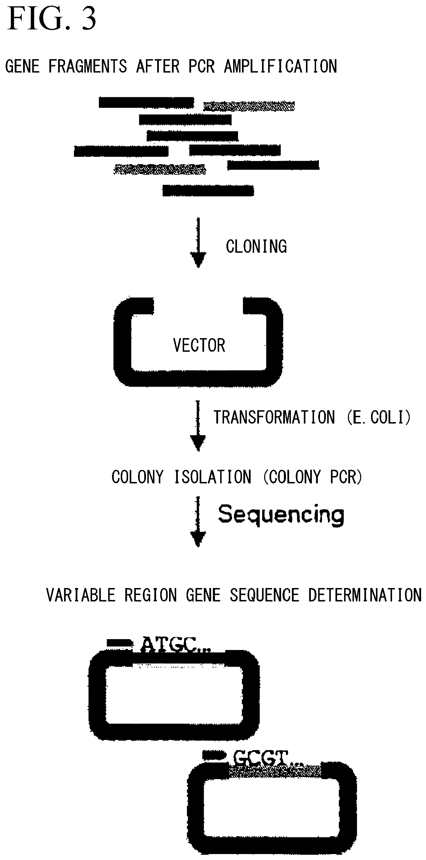

FIG. 3 shows an overview of the method for determining the base sequence after subcloning. After cloning the antibody variable region gene fragment into a vector, it was transformed into a monoclonal cell by introducing the gene into E. coli and transforming it. Clones presumed to be antibody genes were selected by colony PCR using vector sequence-specific primers, and their nucleotide sequences were determined.

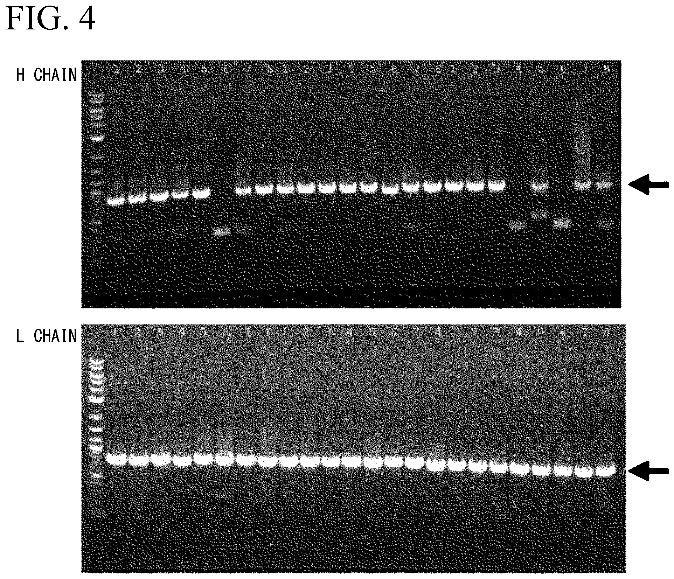

FIG. 4 shows the results of agarose electrophoresis of representative colony PCR products. Amplification products of approximately 0.9 kb (indicated with an arrow in the drawing) were detected for both the H chain and the L chain, and after purification, the base sequences were determined. Quick-Load Purple 1kb Plus DNA Ladder was used as a size marker.

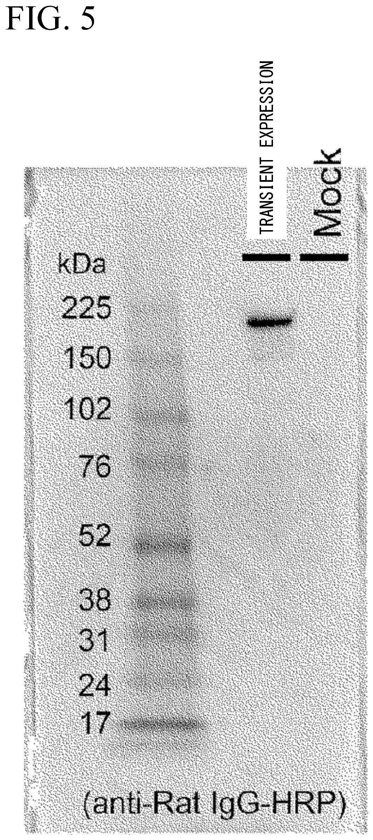

FIG. 5 shows the analysis results of a transiently expressed 92204R antibody by immunoblot. H chain and L chain were coexpressed in CHO-K1 (F) cells, and antibodies in the culture supernatant were subjected to immunoblotting under non-reducing conditions and detected using anti-Rat IgG HRP. Mock: A culture supernatant obtained by culturing gene transfer-negative CHO-K1 (F) cells for the same period was used.

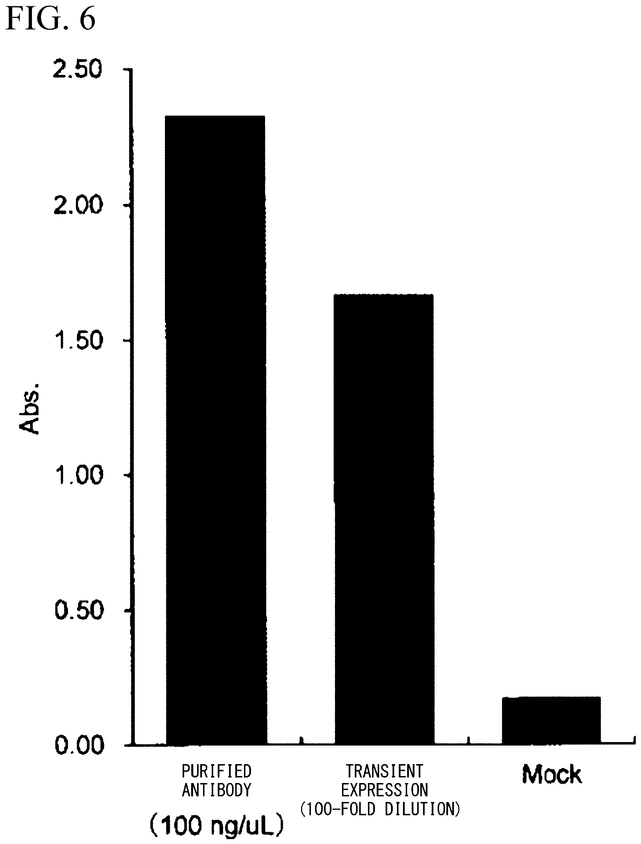

FIG. 6 shows the results of reactivity analysis of a transiently expressed 92204R antibody. The reactivity of the transiently expressed antibody (culture supernatant) to an antigen was analyzed by solid-phase ELISA. Mock: A culture supernatant obtained by culturing gene transfer-negative CHO-K1 (F) cells for the same period was used.

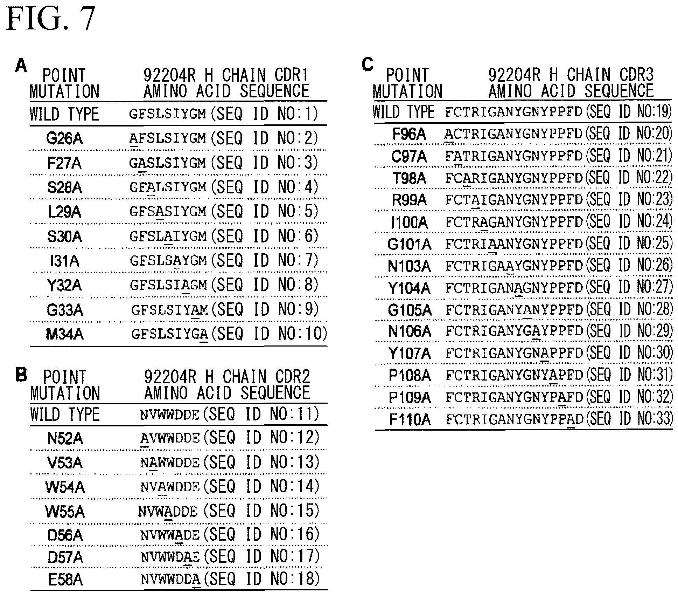

FIG. 7 shows CDR sequences of mutant antibodies with various point mutations added for screening variable regions involved in reaction with antigens. In order to search for regions strongly involved in antigen reactivity, a mutant antibody was prepared, in which amino acids in CDR1 (A), CDR2 (B), and CDR3 (C) of the assumed H chain variable region were replaced with alanine (underlined in the drawing).

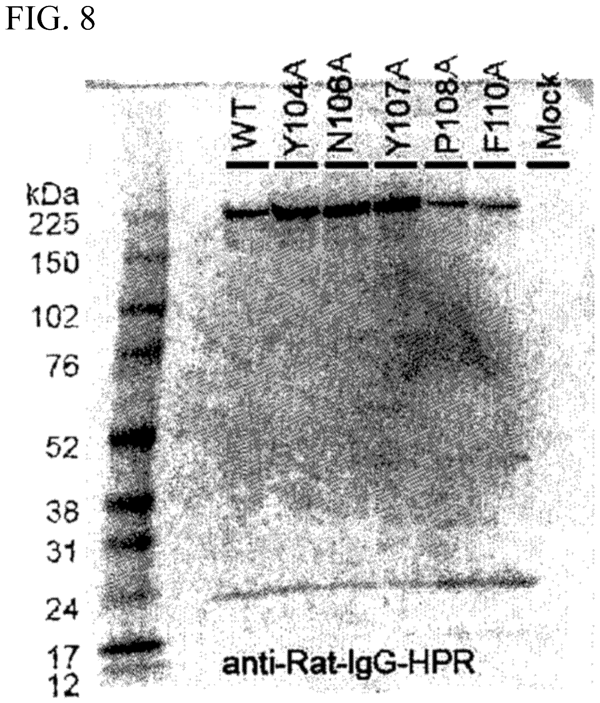

FIG. 8 shows the transient expression results of representative mutant antibodies by immunoblot. H chain and L chain were coexpressed in CHO-K1 (F) cells, and antibodies in the culture supernatant were subjected to immunoblotting under non-reducing conditions and detected using anti-Rat IgG HRP. Mock: A culture supernatant obtained by culturing gene transfer-negative CHO-K1 (F) cells for the same period was used.

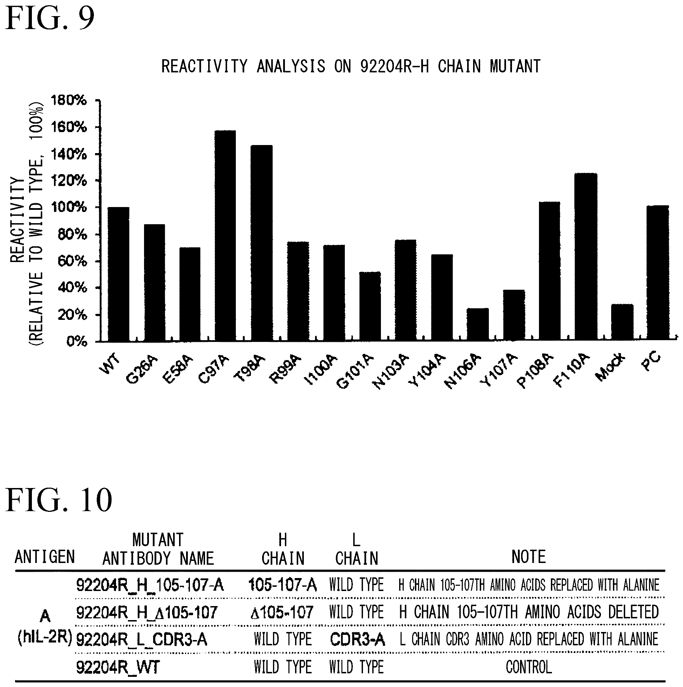

FIG.9 shows the results of analyzing the antigen reactivity of a transiently expressed mutant antibody (culture supernatant) by solid-phase ELISA with respect to the antigen reactivity of typical mutant antibodies. WT: A transiently expressed wild-type antibody, Mock: gene transfer-negative CHO-K1 (F) cells were cultured for the same period. PC: A positive control. A purified antibody derived from ascites fluid was used.
FIG. 10 is a diagram showing the types and modifications of the H chain and L chain of the H chain mutant antibody (H_105-107-A, H Δ105-107) and the L chain mutant antibody (L CDR3-A). Gene fragments for both antibodies were obtained by artificial gene synthesis and cloned into mammalian cell expression vectors.

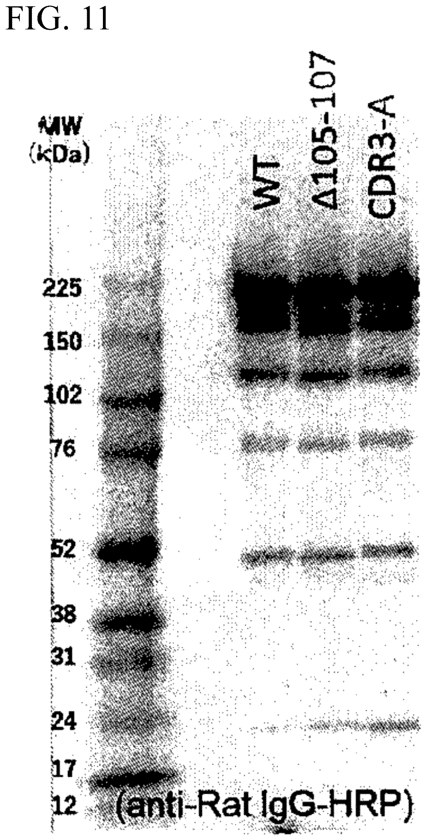

FIG. 11 shows the analysis results of modified antibody IgG by immunoblot. Modified antibody IgG in the culture supernatant transiently expressed by ExpiCHO cells was detected by anti-Rat IgG HRP.

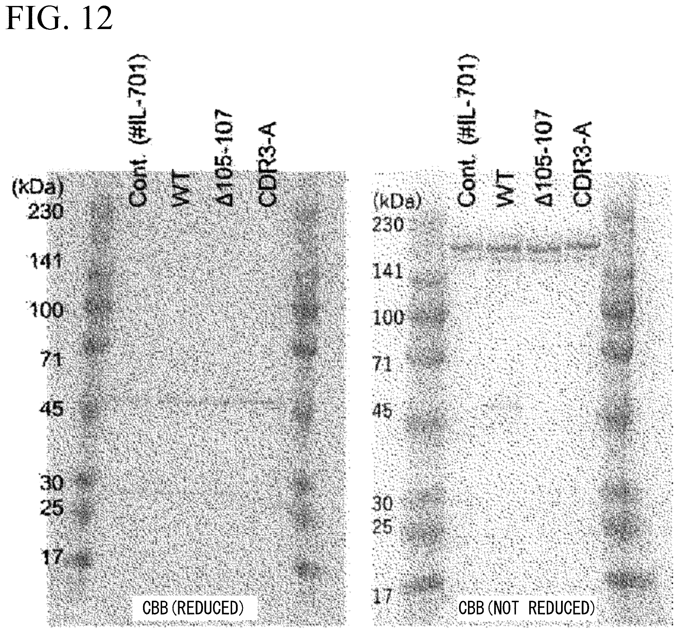

FIG. 12 is a diagram showing the results of purity analysis of purified antibodies. The purified antibodies were subjected to SDS-PAGE under reducing and non-reducing conditions, followed by CBB staining. Cont. (#IL-701): A purified ascites-derived 92204R antibody.

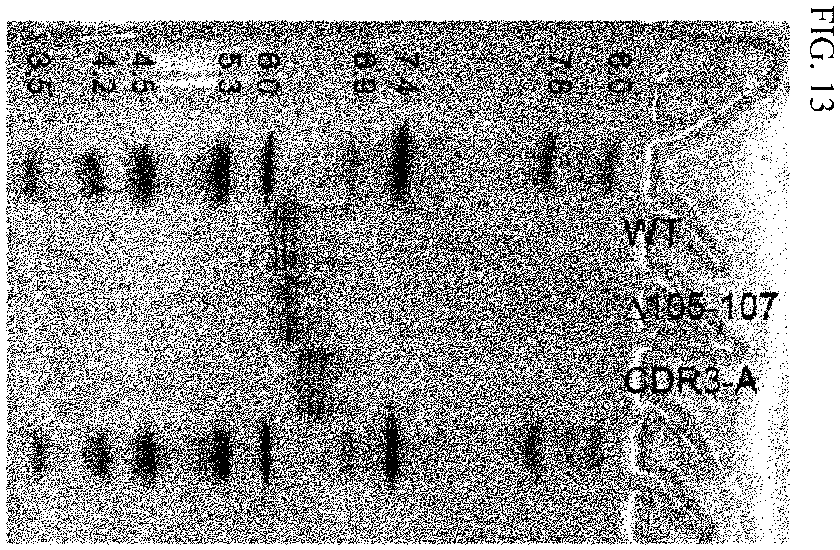

FIG. 13 shows the results of analyzing the isoelectric points of 92204R_H_Δ105-107 antibody and 92204R_L_CDR3-A antibody.

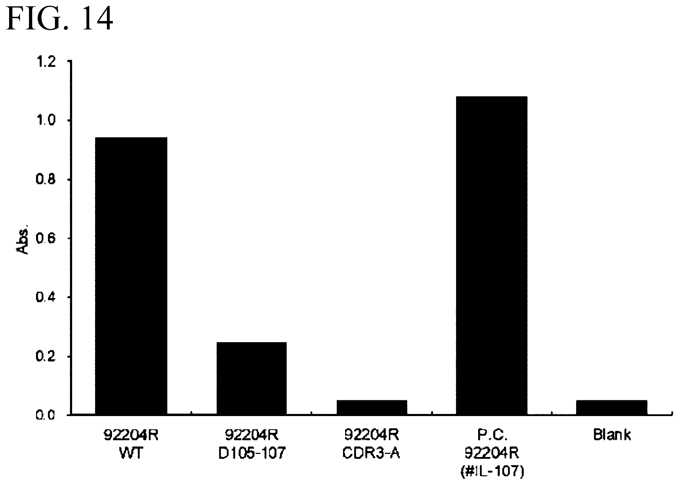

FIG. 14 is a diagram showing the results of reactivity analysis of the mutant antibody of the present invention. Each mutant antibody was prepared at 100 µg/mL, and reactivity to the antigen was evaluated by solid-phase ELISA. Blank: A well without immobilized antigen was used as a negative control.

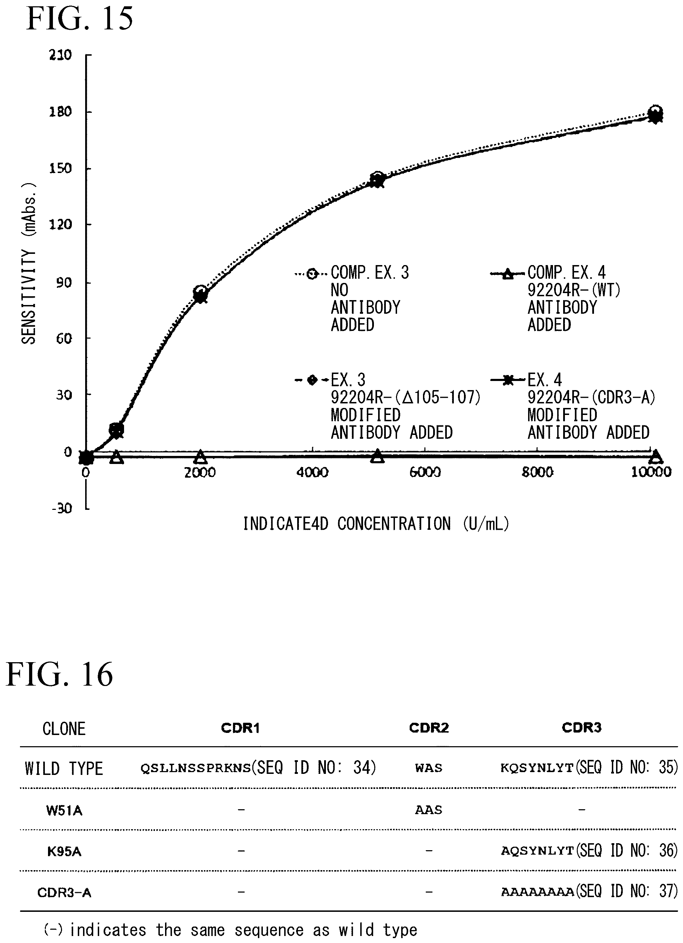

FIG. 15 is a diagram showing the results of verifying the effect of adding a CDR-modified antibody on specific reaction in the LTIA method. The abscissa shows the sIL-2R concentration (U/mL), and the ordinate shows the sensitivity (mAbs.).
FIG. 16 is a diagram showing the modification details of 33236-(W51A) modified antibody, 33236-(K95A) modified antibody, and 33236-(CDR3-A) modified antibody, which are L chain CDR modified antibodies of anti-BNP IgG monoclonal antibody 33236.

### [Description of Embodiments]

### (Modified antibody)

In the method for detecting a target antigen in a sample according to the present invention using a specific antibody that specifically binds to the antigen, the antibody allowed to coexist in order to suppress non-specific reactions refers to a modified antibody in which part or all of the variable region of the L chain and/or H chain of the specific antibody is modified (hereinafter also referred to as "modified antibody of the present invention" or simply "modified antibody"). Modification of part or all of the variable region of the L chain and/or H chain of the specific antibody is not particularly limited as long as at least one amino acid in the variable region is modified, and also encompasses modifying a plurality of amino acids or all of the amino acids. The term "modification" is used to include any mutation such as substitution, deletion, or addition. The plurality of amino acids are two or more amino acids, such as 2, 3, 4, 5, 6, 7, 8, 9, 10, 11, 12, 13 14, 15, 16, 17, 18, 19, 20, etc. Further, examples of preferable ranges include 2 to 20, 2 to 15, 2 to 10, and 2 to 5.

Therefore, the modified antibody of the present invention encompasses any of: an antibody in which part or all of the variable region of the L chain and/or H chain of the specific antibody is replaced with a different amino acid, an antibody in which part or all of the variable region is deleted, and an antibody with one or more amino acid residues added to the variable region. Further, the modified antibody is a modified antibody in which part or all of the variable regions of the L chain and/or H chain of a specific antibody that are particularly largely involved in antigen-antibody reactions (generally CDRs) are modified, or a modified antibody in which part or all of the region related to the three-dimensional structure of CDRs is modified, and it is desirable that the modified region be as small as possible. Examples of the region related to the three-dimensional structure of CDRs include a region adjacent to the N-terminal and/or C-terminal side of the amino acid sequence of each CDR, a region that interacts with the three-dimensional structure of each CDR, and the like.

For example, the modified antibody is preferably a modified antibody in which is modified with respect to part or all of at least one CDR among the six CDRs in the variable regions of the L chain and H chain of the specific antibody, and is not modified at all with respect to at least one CDR different from the modified CDR(s) described above. This is because leaving at least one CDR completely unmodified enables non-specific reactions depending on the sequence of the CDR to be suppressed, and makes it possible to broadly suppress non-specific reactions. Therefore, it is desirable to leave a large number of CDRs unmodified. The number of unmodified CDR may be 1, but is preferably 2 or more, more preferably 3 or more, even more preferably 4 or more, and most preferably 5. Further, other regions of the modified antibody may be the same as or different from those of the specific antibody.

As another embodiment of the modified antibody, for example, the modified antibody may be one with its binding to the target antigen of the specific antibody lowered by modifying, as a region near the CDR related to the three-dimensional structure of the CDR, amino acids near the N-terminus and/or C-terminus of the amino acid sequence of the CDRs, or by modifying amino acids in framework regions involved in the three-dimensional structure of the CDRs. Amino acids near the CDRs include 1 to 5 amino acids from the N-terminus and/or C-terminus of the amino acid sequence of each CDR.

The modified antibody of the present invention is also an antibody that does not compete with the wild-type specific antibody for binding in a reaction with a target antigen when the modified antibody is allowed to coexist with a specific antibody without amino acid modifications (wild-type antibody) in the reaction system.

Specifically, whether the modification is such that the modified antibody does not compete with the wild-type antibody can be evaluated based on the specificity for the target antigen. The modified antibody has a specificity for the target antigen of preferably 50 % or less, more preferably 40 % or less, even more preferably 30 % or less, even more preferably 20 % or less, most preferably less than 10 %, relative to the WT (wild type) antibody. The specificity can be analyzed, for example, by solid-phase ELISA, as shown in the Examples below.

Examples of the modified antibody of the present invention include antibodies in which part or all of the variable region of the L chain and/or H chain of the antibody that binds to the target antigen described below is replaced with a different amino acid, and more specific examples include an antibody in which part or all of the variable region of the L chain and/or H chain of an antibody produced from an anti-sIL-2R antibody-producing hybridoma (accession number: NITE BP-02123) is replaced with a different amino acid; and an antibody in which part or all of the variable region of the L chain and/or H chain of an antibody produced by an anti-BNP antibody-producing hybridoma (accession number: NITE ABP-03799) is replaced with a different amino acid.

Further, the modified antibody may be any type of antibody, such as an intact antibody (full-length antibody) or a fragment antibody, as long as it contains at least a part of the variable region of a specific antibody and can suppress the non-specific reaction of the specific antibody that specifically binds to the target antigen.

Specifically, when the specific antibody is a full-length antibody, the modified antibody may be a full-length antibody or a fragment antibody that contains the CDR of a specific antibody, and is modified at amino acids in the CDR and/or the region near the CDR. Further, when the specific antibody is a fragment antibody, examples of the modified antibody include a fragment antibody in which amino acids in the CDR and/or the region near the CDR of the fragment antibody are modified, and a full-length antibody containing such a modified fragment antibody.

Regardless of whether the specific antibody is a full-length antibody or a fragment antibody, a desired modified antibody can be selected, designed and produced by assuming and identifying the site responsible for the non-specific reactions using protease treatment or fragments derived from individual specific antibodies produced as genetically recombinant proteins.

The modified antibody of the present invention binds to a non-specific factor present in the sample instead of the specific antibody, which makes it possible to broadly suppress non-specific reactions caused by the non-specific factor binding to the specific antibody. Therefore, the modified antibody of the present invention functions as an active ingredient of a non-specific reaction inhibitor. In other words, the modified antibody of the present invention is an antibody that serves as a "decoy" for a specific antibody against a non-specific factor, and can also be said to be an antibody that serves as a "substitute". Antibodies with such effects are included in the modified antibodies of the present invention.

Heretofore, in order to stably measure a target antigen with high sensitivity, it has generally been implemented to create an antibody with higher reactivity with a target antigen by modifying the antibody. However, in the present invention, conversely, a specific antibody that is partially modified and has low reactivity with antigens is utilized to enable non-specific reactions to be effectively suppressed with excellent versatility. That is, the present invention has succeeded in providing a modified specific antibody, a method for suppressing non-specific reactions using the modified antibody, and a detection reagent that utilizes the action of the modified antibody.

The outline of the creation of the modified antibody of the present invention is as follows.

First, in order to obtain genetic information of a specific antibody for a target antigen, the H/L chain gene is cloned, its nucleotide sequence is determined, and the predicted amino acid sequence is identified, followed by analysis of the functional domain.

Next, a search for the modified antibody of the present invention is performed. Modifications are carried out by introducing various mutations into the variable region of the H chain or L chain of the specific antibody using genetic recombination technology. Among the variable regions of the H chain or L chain, CDR regions, neighboring regions adjacent to the N-terminus and/or C-terminus of the amino acid sequence of each CDR, or regions that interact with the three-dimensional structure of each CDR are desirable.

Specifically, the search for the modified antibody of the present invention can be carried out by the following procedure. A CDR-modified antibody in which all amino acids in CDR1, CDR2, and CDR3 are replaced with alanine is prepared, and the CDRs that contribute to binding to the antigen are determined by comparing and evaluating their reactivity to the antigen with that of the wild type. This CDR-modified antibody may also be able to suppress non-specific reactions. Since it is desirable that the modification site be as small as possible, point mutant antibodies are created in which the amino acids in the CDR revealed by the above method were mutated one by one to alanine, and the reactivity of the point mutant antibodies are compared and evaluated with that of the wild type as described above to determine which amino acids are important. The point mutant antibodies may also be able to suppress nonspecific reactions. Since modified antibodies with further reduced reactivity to antigens may be required, modified antibodies in which the amino acids determined above are replaced with amino acids other than alanine, or multiple modified antibodies in which the surrounding amino acids are replaced with alanine, or a modified antibody with the amino acid deleted, or a modified antibody with multiple deletions in the vicinity including the amino acid, or a modified antibody with new amino acids added before and after the amino acid is produced, and the reactivity is similarly compared and evaluated with the wild type so as to select modified antibodies.

Determination of reduced reactivity of the obtained modified antibody candidates to the target antigen is performed by the following method. First, a modified antibody is produced using a transient expression system in animal cells or the like. Next, a modified antibody whose reactivity to the antigen is significantly reduced compared to the wild type is selected by antigen solid-phase ELISA or the like. For the production of this transiently expressed antibody, a cell-free system or an expression system such as E. coli can also be used.

Further, by performing antibody solid-phase ELISA or the like using the selected modified antibodies, it is possible to select modified antibodies whose reactivity with non-specific substances in the sample is equivalent to that of the wild type.

Through these steps, it is possible to obtain a modified antibody that can suppress non-specific reactions without affecting the reactivity of the specific antibody of the present invention with the target antigen.

In the detection method including the step of bringing an antigen in a sample into contact with a specific antibody in the presence of the modified antibody of the present invention, the number of specific antibodies may be one or two or more, and it is sufficient that the modified antibody include a modified antibody against at least one specific antibody among the specific antibodies.

In another embodiment, the specific antibody may include two types of antibodies, i.e., a first specific antibody and a second specific antibody, while the modified antibody may include a modified antibody/antibodies of at least one of the first specific antibody and the second specific antibody.

Further, the specific antibody may include two types of antibodies, i.e., a first specific antibody and a second specific antibody, while the modified antibody may include two or more modified antibodies of either the first or second specific antibody, or the modified antibody may include two types of modified antibodies that are one type of modified antibody of the first specific antibody and one type of modified antibody of the second specific antibody. Each modified antibody may be used alone or two or more types of the modified antibodies may be used in the form of a mixture. Further, the modified antibody may be bound to an insoluble carrier.

In the present invention, the concentration of the modified antibody may be any concentration that does not have a strong influence on the antigen-antibody reaction and can exert the desired non-specific reaction suppressing effect, and can be determined by those skilled in the art as appropriate depending on the types of target antigen samples. The concentration of the modified antibody in the antigen-antibody reaction system varies depending on the reagent composition, but is 0.75 to 750 µg/mL, preferably 3.0 to 450 µg/mL, more preferably 9.0 to 375 µg/mL. However, the concentration is not limited to these examples. Further, the concentration ratio of the modified antibody to the specific antibody in the antigen-antibody reaction system is 0.1 to 140 times, preferably 0.4 to 85 times, more preferably 1.2 to 70 times. However, the ratio is not limited to these examples. For example, in some cases, less than 30 times may be preferred. More specifically, the ratio may be 1 to 29 times, 5 to 20 times, 10 to 15 times, or less than 10 times, e.g., 1 to 9 times, 2 to 7 times, 3 to 6 times, etc. The modified antibody of the present invention exhibits the effect of suppressing a non-specific reaction even at a low concentration ratio to the specific antibody, and can further reduce the influence of the specific antibody on the antigen-antibody reaction. Since the concentration of the modified antibody can thus be kept low, the reagent costs can be suppressed, and the modified antibody has less influence on other components in the measurement system; therefore, the modified antibody can be expected to be used regardless of the type of measurement principle.

With respect to the modified antibody, a single type thereof may be used alone or two or more types thereof may be used in the form of a mixture. Further, the modified antibody may be bound to an insoluble carrier. Further, when two or more modified antibodies are used together, the total concentration of the antibodies may be within the above concentration range.

For allowing the modified antibody to be included in the detection reagent of the present invention in advance, the modified antibody may allowed to be included in the detection reagent in advance so that its concentration in the reaction system falls within the above range.

In addition, as another embodiment of using the modified antibody of the present invention, pre-treating a sample containing the target antigen with the modified antibody in advance can allow the specific antibody to react with a sample from which non-specific factors that could potentially bind to the specific antibody and cause non-specific reactions have been removed by adsorption in advance.

### (Specific antibody)

The specific antibody in the present invention may be any antibody specific to the antigen to be measured, and antibodies such as polyclonal antibodies and monoclonal antibodies (including recombinant antibodies and functional fragments of the antibodies) may be selected. Among these, monoclonal antibodies are preferably used. Further, the specific antibody of the present invention may be an intact antibody (full-length antibody) or a fragment antibody (functional fragment) containing a binding site for the target antigen. The specific antibody of the present invention may be any of those obtained through the immunization process relative to common animals (mice, rats, rabbits, goats, sheep, etc.), as well as antibodies (chimeric antibody, humanized antibody, fully humanized antibody, etc.) whose amino acid sequence has been changed by genetic recombination technology, etc. to that of an animal species different from the animal to be immunized with the immunogen (substance to be measured). Examples of fragment antibodies include F(ab')₂, Fab', and single chain antibody (scFv), which are fragments having antigen-binding activity. These fragment antibodies can be produced by treating the antibody obtained as described above with a proteolytic enzyme (e.g., pepsin, papain, etc.).

The specific antibody in the present invention may be used in either a free form or a bound form, and the form is selected depending on the measurement principle. For example, the specific antibody may be bound to a well-shaped plate or labeled with a labeling substance in the case of enzyme-linked immunosorbent assay (hereinafter abbreviated as ELISA), or may be bound to latex particles in the case of latex immunoturbidimetry (hereinafter sometimes abbreviated as LTIA), or may be bound to colloidal gold particles or to a porous membrane in the case of immunochromatography. Furthermore, in chemiluminescence method, the antibody may be bound to magnetic particles.

When using two or more specific antibodies, for example, one may be bound to an insoluble carrier while the other being free (ELISA, chemiluminescence), or both may be bound to an insoluble carrier (LTIA, immunochromatography).

Further, when two types of specific antibodies are bound to an insoluble carrier, both may be intact antibodies, one of them may be a fragment antibody, or both may be fragment antibodies.

### (Sample)

Examples of samples containing the measurement target antigen in the present invention include blood, serum, plasma, culture supernatant, urine, spinal fluid, saliva, sweat, ascites, or cell or tissue extracts of humans or animals. Examples of the sample in the present invention include not only the specimen itself obtained from a living body but also a specimen having been subjected to pretreatment such as dilution and purification.

### (Target antigen)

In the present invention, various antigens contained in the sample can be measurement target antigens. Examples of target antigens include proteins, peptides, amino acids, glycoproteins, lipids, carbohydrates, glycolipids, nucleic acids, haptens, etc., but are not particularly limited as long as they are substances that can be measured theoretically. More specific examples include CRP (C-reactive protein), Lp(a), MMP3 (matrix metalloproteinase 3), antiphospholipid antibodies, type IV collagen, PSA, BNP (brain natriuretic peptide), sIL-2R (soluble interleukin-2 receptor), insulin, albumin, cystatin C, RF (rheumatoid factor), KL-6, procalcitonin, FDP, D-dimer, SF (soluble fibrin), TAT (thrombin-antithrombin III complex), PAI-1, phenytoin, phenobarbital, carbamazepine, valproic acid, theophylline, etc.

Example of method for causing the modified antibody of the present invention to be present in an antigen-antibody reaction system in order to bring the antigen in a sample into contact with the specific antibody include a method that uses the modified antibody as one of the reagent components of a detection reagent, and a method that adds the modified antibody to a sample diluent or a pretreatment solution.

For example, when the detection reagent is a liquid reagent, the antigen-antibody reaction system refers to the liquid phase in which the sample and the liquid detection reagent are mixed and an immune reaction is performed.

For example, in the case of the LTIA method, a sample may be mixed with an LTIA detection reagent containing the modified antibody, or a sample may be mixed in advance with a pretreatment solution containing the modified antibody and then mixed with the detection reagent.

Further, in the case of ELISA, a sample may be mixed in advance with a pretreatment solution containing the modified antibody and then dropped onto a well-shaped plate, or a sample may be mixed with a solution containing the modified antibody and the specific antibody and then dropped into a well-shaped plate.

Also in the case of chemiluminescence reagents, a sample may be mixed in advance with a pretreatment solution containing the modified antibody and then mixed with a detection reagent, or the detection reagent (for example, a solution containing the specific antibody, magnetic particles, etc.) may contain the modified antibody.

Furthermore, when an immune reaction is performed on a solid phase, such as in an immunochromatography method, the antigen-antibody reaction system refers to the solid phase where the immune reaction between a liquid sample and the specific antibody is performed. In this case, the sample may be mixed in advance with a pretreatment solution containing the modified antibody and then dropped onto the immunochromatography test piece, or the modified antibody may be kept dry at the sample supply site so that when the sample is dropped, the modified antibody is dissolved to develop a solid phase and thereby allowed to be present in the reaction system.

### (Detection method)

The present invention is an immunological detection method in which an antigen to be detected in a sample is detected using a specific antibody, in which an immune reaction is performed in the presence of a modified antibody.

Immunological detection methods can be further divided into homogeneous methods and heterogeneous methods.

The homogeneous method is a measurement method that specifically detects the reaction progressing with a target substance in a mixed solution (reaction solution) of a sample and a reagent solution without performing B/F (bond/non-bond) separation. The heterogeneous method is a measurement method in which excess components not involved in the measurement reaction are washed and removed by a B/F separation operation, and then the main reaction is allowed to proceed and detected.

While the heterogeneous method has the problem that it involves a washing step and, hence, requires many steps and takes time to complete measurement, it has the advantage of being relatively less susceptible to the effects of non-specific reactants. On the other hand, while the homogeneous method does not go through a washing step and, hence, tends to be more susceptible to non-specific reactions, this method is widely favored in the field of clinical diagnosis because it is simple with fewer steps and the time required for measurement is short.

Examples of the homogeneous method include a measurement method using immunoagglutination (TIA) and immunochromatography (lateral flow method, flow-through method). TIA is a method for qualitatively or quantitatively detecting an analyte (substance to be measured) in a sample based on the degree of agglutination of an immune complex formed by cross-linking of an analyte by a specific antibody such as an antibody. Particularly, LTIA, which uses latex particles as an insoluble carrier to amplify the agglutination signal, is a highly versatile measurement that is applied to a variety of test items as it is suitable for optical detection and is easy to automate.

Examples of the heterogeneous method include an ELISA method using a well-shaped plate and a chemiluminescence method.

Although the present invention can utilize any of the above immunological detection methods, the homogeneous method, which is relatively susceptible to non-specific reactions, is expected to enjoy the effects of the present invention more than other methods.

In the present specification, the term "detection" is used in the broadest sense, including verification and/or quantification of the existence of a target object, and also encompasses the concept of "measurement" indicating quantification.

### (Detection reagent)

The detection reaction of the present invention is carried out using a detection reagent, and the reagent contains a specific antibody against the target antigen, which is the main component of the reaction, as well as a variant of the specific antibody. Further, the reagent may include insoluble carriers.

In addition, the detection reagent may contain components that buffer or adjust the pH, ionic strength, osmotic pressure, etc. of the sample, such as acetic acid, citric acid, phosphoric acid, Tris, glycine, boric acid, carbonic acid, and Good's buffer, or their sodium salts, potassium salts, calcium salts, etc. Further, a polymer such as polyvinylpyrrolidone or phospholipid polymer may be included as a component that enhances agglutination formation. In addition, as a component to control the formation of agglutination, the reagent may contain one or a combination of commonly used components such as proteins, amino acids, sugars, metal salts, surfactants, reducing substances, and chaotropic substances.

### (Insoluble carrier)

The insoluble carrier used in the present invention may be any carrier as long as it can support a specific antibody against the desired target component, and known particles can be used. Such particles are particularly referred to as insoluble carrier particles. The insoluble carrier particles are preferably latex particles made of polymeric materials such as polystyrene, but inorganic particles such as metal colloids, silica, and carbon may also be used as the insoluble carrier particles of the invention, depending on the method of supporting specific binding partners for the target antigen.

The size of the insoluble carrier particles is appropriately selected from the range of 0.04 to 1 µm, in consideration of the optical measurement method used (e.g., turbidimetry for measuring transmitted light, nephelometric method for measuring scattered light, etc.) to obtain the desired measurement sensitivity, measurement range, etc. In optical measurement using an automatic analyzer, an average particle size of 0.1 to 0.4 µm is commonly used, but the particle size is not limited to this example.

The average particle size of the particles for immunoassay can be determined using a particle size distribution analyzer, a transmission electron microscope image, etc. The concentration of the immunoassay particles in the reagent solution can be appropriately selected, for example, from the range of 0.0001 mg/mL to 10 mg/mL, depending on the particle size of the immunoassay particles used and the overall design of the measurement system.

When latex particles are used as the insoluble carrier particles of the present invention, the synthetic polymer forming the latex particles is not particularly limited, but examples thereof include polystyrene, vinylnaphthalene, styrene-styrene sulfonate copolymer, methacrylic acid polymer, acrylic acid polymers, itaconic acid polymers, and styrene-hydrophilic carboxy monomer copolymers (e.g., styrene-methacrylic acid copolymers, styrene-acrylic acid copolymers, styrene-itaconic acid copolymers, etc.)

An immunoagglutination method employing latex particles as insoluble carrier particles is particularly referred to as latex immunoturbidimetry (LTIA, described below).

A specific antibody against a target antigen can be bound to and supported on latex particles by a known method such as a physical adsorption method, a chemical bonding method, or a combination thereof.

It is preferable that multiple types of specific antibodies are supported on the latex particles so as to form a sandwich. However, if the target antigen is a multivalent antigen, only one type of specific antibody may be used. For example, when the specific antibody is a monoclonal antibody, a combination of multiple monoclonal antibodies with different recognition sites is often used.

Further, if it is necessary to perform treatment to suppress natural agglutination of latex particles or non-specific reactions, etc., the carrier may be subjected to blocking treatment (masking treatment) by treating the surface of latex particles by a known method such as contacting and coating with a protein such as bovine serum albumin (BSA), casein, gelatin, ovalbumin or its salt, a surfactant, or skim milk powder, etc. The non-specific reaction inhibitor containing the modified antibody of the present invention can be expected to be effective against non-specific reactions that cannot be suppressed even after such general non-specific reaction suppression treatment.

### (Latex immunoturbidimetry)

Latex immunoturbidimetry (LTIA method), which is one of the immunological measurement methods of the present invention, is explained below. Methods for measuring a target substance to be measured using the LTIA method can be broadly classified into two types.

The first method is to react latex particles to which a specific antibody against the target substance is bound with the target substance to form a sandwiched immune complex, and to measure the target substance from the degree of agglutination of the latex particles accompanying the formation of the immune complex.

The second method is to add proteins, etc. to which multiple target substances or their analogs (including fragments thereof) are bound into the reagent, and to allow these to compete with the target substances in the sample to inhibit the formation of immune complexes between the target substances contained in the reagent and latex particles bound with the specific antibodies against the target substances, and measure the target substances (antigens) from the degree of inhibition of agglutination of the latex particles due to inhibition of immune complex formation.

Specific examples of the steps to be taken are as follows.
(1) A step of bringing the sample into contact with the modified antibody in a liquid phase.
(2) A step of adding latex particles bound with the specific antibody into a liquid phase to allow the resulting to contact the specific antibody simultaneously with or after the step (1).
(3) A step of measuring the agglutination reaction between the target substance and the latex particles.

In this context, the step (3) means a step of measuring an agglutination reaction between the target antigen and the specific antibody-bound latex particles in the middle of the step (2) or after the step (2) without going through the washing and separation steps.

LTIA can measure a target substance by optically or electrochemically observing the degree of the occurred agglutination. Examples of optical observation methods include methods of measuring scattered light intensity, absorbance, or transmitted light intensity with an optical instrument (end point method, rate method, etc.). The measured values such as absorbance obtained by measuring the sample are compared with the measured values such as absorbance obtained by measuring the standard substance (a sample with a known concentration of the substance to be measured), to calculate the concentration (quantitative value) of the substance to be measured. In this context, the measurement of the absorbance of transmitted light, scattered light, etc. may be a one-wavelength measurement or a two-wavelength measurement (difference or ratio between two wavelengths). The measurement wavelength is generally selected from 500 nm to 900 nm.

Detection of the measurement target antigen in the sample of the present invention may be performed manually or using a device such as a measuring device. The measuring device may be a general-purpose automatic analyzer or a dedicated measuring device (dedicated machine). Further, this measurement is preferably carried out by a method including a plurality of operational steps, such as a two-step method (two-reagent method).

### (Reagent configuration)

The detection reagent of the present invention is composed of one test solution or a plurality of test solutions of two or more test solutions. Examples of multiple test solutions include test solutions composed of a buffer solution for the purpose of adjusting the target antigen to a suitable concentration for measurement or adjusting the environment for antigen-antibody reactions, and test solutions containing specific antibody-binding particles, etc. In the present invention, the modified antibody of the specific antibody may include any or all of the constituent reagents as long as the effect of suppressing non-specific reactions originating from the sample can be obtained in the mixed liquid state during detection and does not affect the stability of the constituent reagents.

In the case of a detection reagent that uses latex particles as an insoluble carrier, an example thereof is a detection reagent that includes a first reagent containing a buffer solution and a second reagent containing latex, one or both of which contains the modified antibody. Further, the sample pretreatment solution may contain the modified antibody. The concentration of the modified antibody in each constituent reagent may be adjusted to the concentration set for the antigen-antibody reaction system in the mixed state of the reagent and the sample during measurement, and varies depending on the configuration of each detection reagent.

Descriptions are presented above on detection/measurement methods and reagent configurations with a focus on LTIA, but descriptions are presented below also on other detection/measurement methods that utilize the modified antibody of the present invention to suppress non-specific reactions in the antigen-antibody reaction system.

### <ELISA method>

The ELISA method is a method that utilizes a combination of various antigen-antibody reactions and finally incorporates an enzyme-labeled antigen or antibody into the reaction system to detect enzyme activity. For detection of enzyme activity, a substrate whose absorption spectrum changes depending on the reaction is used, and direct methods, indirect methods, sandwich methods, competitive methods, etc. are used depending on the combination of antigen-antibody reactions.

The reagents used when the detection method of the present invention is a sandwich ELISA method are illustrated below.
(a) Insoluble carrier having bound thereto a specific antibody that reacts with the measurement target substance.
(b) Specific antibody labeled with a labeling substance and reacts with the target antigen.

As the insoluble carrier (a), a well-shaped plate is preferable, and the labeling substance can be appropriately selected and used. The specific antibody bound to the insoluble carrier captures the target antigen in the solution containing the sample and forms a complex on the insoluble carrier.

The specific antibody labeled with the labeling substance (b) binds to the captured target substance to form a sandwich with the aforementioned complex. By measuring the amount of the labeling substance using a method suitable for the labeling substance, the amount of the target substance in the sample can be determined. Specific methods such as a method for binding a specific antibody to an insoluble carrier and a method for binding a specific antibody to a labeling substance are not particularly limited and may be any method well known to those skilled in the art.

In the above ELISA method, the modified antibody of the present invention can be allowed to present in the reaction system by, for example, adding the antibody to a sample diluent, a pretreatment solution, or the like, or adding the antibody to a solution in which an antigen-antibody reaction is performed.

### <Immunochromatography method>

An explanation is given below on the reagent composition (test piece composition) when the principle of the detection method of the present invention is immunochromatography.

Immunochromatography test piece;
When the specific antibody is used, the test piece is one including a sheet-like insoluble carrier such as a porous membrane, on which the following sites are provided in the direction of development of the solution containing the sample: "1. sample supply site", "2. a site for holding a labeled specific antibody (labeled antibody-holding site), and "3. a site at which a specific antibody for capturing a complex formed by a labeled antibody and a specific antibody is bound (capture antibody site) ".

In the immunochromatography method, when a predetermined amount of a sample containing at least the above-mentioned test piece and containing the target antigen is added to the sample supply site, the sample enters the label-holding site by capillary action, and the target antigen and the labeled specific antibody are bonded to form a complex. When the complex develops the membrane and enters the capture antibody site, it is captured by a specific antibody (capture antibody) bound to the membrane, and a complex of capture specific antibody-target antibody-labeled specific antibody is formed. Then, the measurement target substance can be detected by detecting the label by any method (for example, in the case of a visualizable label such as gold colloid, its agglutination image, or in the case of an enzyme, a color reaction by adding a substrate).

In the present immunochromatography method, the modified antibody of the present invention can be allowed to be present in the reaction system by addition to a sample diluent, a pretreatment solution, etc., or retention in a dry state in a sample supply site or label holding site for a test strip.

### <Chemiluminescence method>

An explanation is given below on the reagent composition when the principle of the detection method of the present invention is chemiluminescence.

The measurement method using chemiluminescence is a method in which magnetic particles bound with an antigen or antibody are brought into contact with the target antigen and a labeled antibody to form a complex which is, then, separated from the unreacted labeled antibody, and a luminescent reagent is added to measure the amount of light emitted. When an enzyme is used as a label, the method is called a chemiluminescent enzyme immunoassay (CLEIA method). Further, the method of measuring luminescence intensity through an electrochemical reaction using a metal complex such as a ruthenium pyridine complex as a label is called an electrochemiluminescence immunoassay (ECLIA method). Furthermore, the method using a chemiluminescent substance as a label is called a chemiluminescence immunoassay (CLIA method).

The reagents used when the detection method of the present invention is the CLEIA method are illustrated below.
(a) Magnetic particles having bound thereto a specific antibody that reacts with the target antigen.
(b) Specific antibody that is enzyme-labeled and reacts with the target antigen.
(c) Luminescent reagent.

The specific antibody bound to the magnetic particles captures the target antigen in the solution containing the sample and forms a complex. The specific antibody labeled with an enzyme labeling substance binds to the captured target antigen to form a sandwich with the aforementioned complex. The target antigen in a sample can be measured by reacting an enzyme labeling substance with a luminescent reagent and measuring the amount of luminescence.

In the above CLEIA method, the modified antibody of the present invention can be allowed to present in the reaction system by, for example, adding the antibody to a sample diluent, a pretreatment solution, or the like, or adding the antibody to a solution in which an antigen-antibody reaction is performed.

### (Method for suppressing non-specific reactions/non-specific reaction inhibitors)

Some components contained in the biological sample often cause agglutination to occur in immunoassay particles having bound thereto specific antibodies against the target antigen that is not supposed to occur (positive measurement error), or prevent aggregation that is supposed to occur (negative measurement error). These are called non-specific reactions and are known to cause various measurement errors.

In the present invention, suppressing non-specific reactions means (passively) acting on the factor (non-specific factor) that causes the above-mentioned non-specific reactions in the sample, and suppressing the influence of reactions other than the specific reaction on detection while acting as a decoy for the specific antibody. The present invention can suppress non-specific reactions by performing an immune reaction in the presence of a modified antibody of a specific antibody against a target antigen. Therefore, the modified antibody of the present invention is also an active ingredient of a non-specific reaction inhibitor.

### (Reagent kit)

The reagent kit of the present invention is characterized in that the kit includes at least a specific antibody for a target antigen and a variant of the specific antibody. As regards the kit configuration, examples of components other than reagents involved in immunological detection include a sample diluent, a sample extract, etc., and the modified antibody of the present invention can be contained in one or more of these.

Typically, the kit contains the reagents (1) and (2), wherein at least one of the reagents (1) and (2) comprise a modified antibody in which part or all of variable region of a L chain and/or H chain of the specific antibody is modified:
(1) a reagent containing a buffer solution, and
(2) a reagent containing the specific antibody.

Examples of reagents containing the specific antibody include reagents in which the specific antibody is bound to an insoluble carrier such as latex.

As regards the kit configuration, examples of components other than mentioned above includes instructions for use and sample collection tools (collection pipette, syringe, cotton swab, filtration filter, etc.).

### (Other reagent components)

The detection reagent of the present invention may contain a polymer such as polyethylene glycol, polyvinylpyrrolidone, phospholipid polymer, etc. as a component that enhances the formation of agglutination of insoluble carrier particles. In addition, as a component to control the formation of agglutination, the reagent may contain one or a combination of commonly used components such as proteins, amino acids, sugars, metal salts, surfactants, reducing substances, and chaotropic substances.

### EXAMPLES

Hereinbelow, a part of the present invention is explained in detail while providing examples of the non-specific reaction inhibitor containing the modified antibody of the present invention, the detection method using the same, the detection reagent, and the method for suppressing a non-specific reaction, which however should not be construed as limiting the present invention.

### [Test Example 1] Preparation of CDR modified antibody

### 1. Analysis of antibody variable region gene sequences

Antibody variable region gene sequences were analyzed using general molecular and cell biological techniques (FIG 1). Specifically, RNA was extracted from approximately 1 million cultured anti-sIL-2R antibody-producing hybridomas (accession number: NITE BP-02123), and full-length cDNA was obtained by reverse transcription and template switching. Using this cDNA as a template, genes for the variable region portions of the H chain and L chain were amplified by RT-PCR (FIG. 2). The amplified sequence was inserted into a vector and E. coli was transformed. The transformed E. coli was cultured, about 12 colonies were picked up, and the cloned sequences were amplified by colony PCR (FIGs. 3 and 4). After purifying this PCR amplification product, sequence information was obtained by direct sequencing, and those without disruption of the translation frame were analyzed. Finally, the results are combined with the 5' end signal sequence estimated using a public database and the known constant region sequence to determine the gene sequences of the H chain and L chain of the anti-sIL-2R monoclonal antibody (92204R antibody) produced from the anti-sIL-2R antibody-producing hybridoma.

### 2. Equivalence analysis of recombinant antibodies

The obtained H chain and L chain genes were transiently expressed in mammalian cells, and their reactivity was analyzed to find whether they were equivalent to the hybridoma-derived 92204R antibody. Specifically, the H chain and L chain genes were cloned into a mammalian cell expression vector by artificial gene synthesis, and the genes were introduced into CHO cells to transiently express IgG in the culture supernatant (FIG. 5). This culture supernatant was collected and its reactivity to the antigen was analyzed by solid-phase ELISA. As a result, it was confirmed that it had the same reactivity as the purified antibody (FIG. 6).

Therefore, it was confirmed that the obtained H chain and L chain genes were genes forming a hybridoma-derived antibody. For the solid-phase ELISA, 50 ng of hIL-2Ra (R&D Systems, Cat.# 2232A025/CF) was immobilized and used as an antigen.

### 3. Screening of modified antibodies

H chain CDR mutant antibody genes were obtained by synthesizing a gene sequence in which alanine was substituted for the complementarity determining region (CDR) of an antibody H chain estimated from a public database. These are incorporated into a mammalian cell expression vector to prepare a mutant antibody expression vector (FIGs. 7A, B, and C).

Next, in the same manner as described above, the resulting was transiently expressed in CHO cells, IgG in the culture supernatant was detected (FIG. 8), and the reactivity to the antigen was further analyzed by solid-phase ELISA. As a result, a mutant in which the reactivity was greatly reduced was observed in the point mutant antibody of H chain CDR3 (FIG. 9). Therefore, it was presumed that the amino acid around the 106th position in CDR3 is mainly involved in the antigen recognition/binding of the 92204R antibody.

### 4. Production of modified antibodies

As the above results suggested that the amino acids around the 106th position in CDR3 are strongly involved in the antigen recognition/binding of the 92204R antibody, a multiple mutant antibody (105-107-A) including amino acids around the 106th amino acid and a deletion antibody (Δ105-107) were prepared as candidates for the modified antibody of the present invention. Further, an antibody in which all amino acids in L chain CDR3 were replaced with alanine was also prepared (FIG. 10), and transient expression and reactivity analysis were performed. (Also with respect to an antibody in which all amino acids in H chain CDR3 were replaced with alanine, transient expression was attempted after the expression vector was prepared, but IgG conversion could not be confirmed, and hence it was not used in the subsequent experiments).

As a result of analyzing the reactivity to the antigen by solid-phase ELISA in the same manner as described above (FIG. 11), it was revealed that the reactivity was significantly decreased in Δ105-107 and L_CDR3-A; therefore, it was decided that the antibodies were to be used in the subsequent experiments as modified antibodies. First, these modified antibodies were expressed using the ExpiCHO cell expression system (ThermoFisher), and the existence of IgG in the culture supernatant was confirmed by immunoblotting. Since a sufficient amount of IgG was confirmed, including wild type, these antibodies were purified using a Protein G column. When the purity of the purified antibodies was analyzed by CBB (Coomassie Brilliant Blue) staining, it was confirmed that it was equivalent to known purified antibodies (FIG. 12); therefore, it was decided to further analyze the isoelectric point.

As a result of isoelectric focusing, H_Δ105-107 had an isoelectric point equivalent to that of the wild type, and L_CDR3-A had a slightly higher isoelectric point (both at a pH range of from 6.0 to 6.9). Thus, it was revealed that there was no significant change in the isoelectric point due to the modification (FIG. 13). Finally, it was decided to determine the reactivity of the purified antibodies using solid-phase ELISA. Antibody solutions were prepared by diluting the purified antibodies 10 times, 100 times, 1,000 times, ..., 10,000,000 times, starting from 1 µg/mL, and the reactivity was analyzed by solid-phase ELISA.

As a result, positive subject (PC) 92204R (#IL-107), which was a purified antibody derived from ascites, and antibody 92204R, which was produced by genetic recombination of the antibody, had the same antigen reactivity, but at a concentration of 100 ng/mL, H_D105-107 showed a reactivity of about 23 % compared to the positive control (PC), and L_CDR3-A showed a reactivity of about 5 % of PC (equivalent to Blank) (FIG. 14). In addition, in this test, the positive subject, which was an unmodified antibody, was a purified antibody derived from ascites, which indicated that the decrease in reactivity of the modified antibody compared to the positive subject was not a decrease in antibody reactivity due to transient expression, but was due to the amino acid alteration itself.

Based on the above results, the modified antibodies prepared above were considered to have a function that might suppress non-specific reactions in sample measurements.

### [Test Example 2] Verification of non-specific agglutination suppressing effect of antibody-bound latex by adding CDR-modified antibodies (LTIA method)

A test described below was implemented to evaluate the degree of agglutination between components in a sample and one type of antibody-bound latex using a buffer solution to which the CDR-modified antibody of the present invention and an unmodified antibody were added.

Since the antigen sIL-2R is not a multivalent antigen with multiple identical epitopes in its molecule, one type of antibody-bound latex alone cannot generally cause agglutinations even if the sIL-2R in the sample and the antibody-bound latex react, so that the absorbance does not change. Therefore, the absorbance change that occurs in the evaluation method shown in this test example can be considered to be due to nonspecific agglutination of the nonspecific factor and the antibody-bound latex in the sample.

### 1. Measurement reagent

(1) Preparation of first reagent
   [Comparative Example 1] A first reagent was prepared according to the method described in JP-A-2017-181377.
   [Comparative Example 2] 92204R-(WT) antibody (unmodified antibody) was added to the first reagent of Comparative Example 1 at a concentration of 100 µg/mL.
   [Example 1] 92204R-(Δ105-107) modified antibody was added to the first reagent of Comparative Example 1 at a concentration of 100 µg/mL.
   [Example 2] 92204R-(CDR3-A) modified antibody was added to the first reagent of Comparative Example 1 at a concentration of 100 µg/mL.
(2) Preparation of second reagent containing one type of antibody-bound latex

A 92204R antibody-bound latex was prepared according to the method of the second reagent (ii) of Test Example 1 described in JP-A-2017-181377. With 5mM MOPS buffer (pH 7.0), a second reagent was prepared so as to exhibit an absorbance of 2.5Abs. /mL at a wavelength of 600 nm.

### 2. Sample

Samples used are samples (control samples: samples 1 to 5) that do not change in absorbance with only one type of antibody-bound latex (92204R antibody-bound latex), and samples (non-specific agglutination samples: samples 6 to 9) that exhibit a non-specific reaction and change in absorbance only with the 92204R antibody-bound latex.

### 3. Measurement procedure

The first reagent and the second reagent were combined, and the degree of nonspecific agglutination between each sample and the 92204R antibody-bound latex alone was evaluated using a Hitachi 7180 automatic analyzer. Specifically, 120 µL of the first reagent was added to 5.6 µL of the sample and kept at 37 °C for 5 minutes, and then 40 µL of the second reagent was added and stirred. The absorbance change associated with agglutination formation was then measured over a period of 5 minutes at a main wavelength of 570 nm and a sub wavelength of 800 nm.

### 4. Measurement result

The measurement results are shown in Table 1.

**[Table 1]**

| | | Agglutination degree (Change in absorbance) (mAbs.) | | | |
|---|---|---|---|---|---|
| Type | Sample No. | Comp.Ex. 1 | Comp.Ex.2 92204R-(WT) antibody added | Example 1 92204R-(Ll 105-107) modified antibody added | Example 2 92204R-(CDR3-A) modified antibody added |
| | | No antibody added | | | |
| Control sample | 1 | -1.1 | -1.4 | --1.0 | -0.9 |
| | 2 | -1.3 | -0.8 | -0.9 | -0.9 |
| | 3 | -2.1 | -2.0 | -1.8 | -1.6 |
| | 4 | -0.7 | -1.1 | -1.4 | -0.8 |
| | 5 | -1.8 | -1.5 | -0.6 | -1.3 |
| Non-specific agglutination sample | 6 | 5.6 | -0.7 | -0.5 | -0.4 |
| | 7 | 2.4 | -0.5 | -0.3 | 0.1 |
| | 8 | 9.3 | -0.3 | 0.0 | 0.2 |
| | 9 | 18.3 | 1.1 | 1.1 | 2.0 |

### 5. Observation

The effects of the present invention were studied from the results of Comparative Examples 1 and 2 and Examples 1 and 2.
(1) From the results of Comparative Example 1, in samples 1 to 5, the change in absorbance of 92204R antibody-bound latex alone was 2.0 mAbs. or less, and no agglutination was observed. On the other hand, in samples 6 to 9, the change in absorbance of the 92204R antibody-bound latex alone was 2.0 mAbs. or more, and nonspecific agglutination was observed.
(2) When using the first reagent having 92204R-(WT) added thereto, the change in absorbance of the 92204R antibody-bound latex was significantly reduced (2.0 mAbs. or less) in samples 6 to 9, and non-specific agglutination was suppressed. No difference was observed in the degree of agglutination in control samples 1 to 5. This indicates that the non-specific factors contained in samples 6 to 9 acted as a decoy by reacting with the 92204R-(WT) antibody added to the first reagent, and suppressed the non-specific agglutination of the non-specific factors and the 92204R antibody-bound latex after the addition of the second reagent (Comparative Example 2).
(3) When using the first reagent having the 92204R-(Δ105-107) modified antibody added thereto, as in Comparative Example 2, the change in absorbance of the 92204R antibody-bound latex was significantly reduced (2.0 mAbs. or less) in samples 6 to 9, and non-specific agglutination was suppressed. No difference was observed in the degree of agglutination in control samples 1 to 5 (Example 1).
(4) When using the first reagent having the 92204R-(CDR3-A) modified antibody added thereto, as in Comparative Example 2 and Example 1, the change in absorbance of the 92204R antibody-bound latex was significantly reduced (2.0 mAbs. or less) in samples 6 to 9, and non-specific agglutination was suppressed. No difference was observed in the degree of agglutination in control samples 1 to 5 (Example 2).
(5) Thus, in the immunoassay method, the CDR-modified antibody of the present invention, when allowed to be present in the antigen-antibody reaction system, acts as a decoy and can thereby suppress non-specific agglutination of the antibody-sensitized latex caused by non-specific factors in the sample.

### [Test Example 3] Verification of the effect of addition of CDR modified antibody on specific reaction (LTIA method)

The concentration of sIL-2R in the sample was measured using a LTIA reagent to which the modified antibody of the present invention was added.

### 1. Measurement reagent

(1) First reagent
   [Comparative Example 3] The first reagent shown in Comparative Example 1 was used.
   [Comparative Example 4] The first reagent shown in Comparative Example 2 was used.
   [Example 3] The first reagent shown in Example 1 was used.
   [Example 4] The first reagent shown in Example 2 was used.
(2) Second reagent

A second reagent prepared according to the method of Test Example 1 described in JP-A-2017-181377 was used. The specific antibody concentration in the reagent was 20 to 30 µg/mL.

### 2. Sample

The following samples were subject to measurement as samples with known concentrations.
(1) Physiological saline solution (OTSUKA NORMAL SALINE).
(2) IL-2R Calibrator N (Sekisui Medical Co., Ltd.) prepared to have the concentrations shown in Table 2.

### 3. Measurement procedure

The first reagent and the second reagent were combined, and the sample with known concentration (physiological saline, IL-2R Calibrator N) was measured using a Hitachi 7180 automatic analyzer. Specifically, 120 µL of the first reagent was added to 5.6 µL of the sample and kept at 37 °C for 5 minutes, and then 40 µL of the second reagent was added and stirred. The absorbance change associated with agglutination formation was then measured over a period of 5 minutes at a main wavelength of 570 nm and a sub wavelength of 800 nm.

### 4. Measurement result

The measurement results are shown in Table 2 and FIG. 15.

**[Table 2]**

| | | Rl-added antibody (100 µg/mL) | | | |
|---|---|---|---|---|---|
| IL-2R indicated concentration | | Comp.Ex. 3 | Comp.Ex.4 92204R-(WT) | Example 3 92204R-(LI 105-107) | Example 4 92204R•(CD R3·A) |
| | | No antibody added | antibody added | modified antibody added | modified antibody added |
| Physiol ogical saline | 0 U/ml | -3.0 | -2.3 | -2.6 | -2 6 |
| Cal① | 509 U/ml | 12,1 | -2.4 | 10.3 | 11.1 |
| Cal② | 2014 U/ml | 84.9 | -2.5 | 82.0 | 82.0 |
| Cal③ | 5150 U/mL | 145.0 | -2.0 | 143.4 | 143.3 |
| Cal④ | 10089 U/ml | 179.8 | -2.6 | 176.6 | 177.6 |
| | Influence on specific reaction | | Strong inhibition | No influence | No influence |

| | | | | | |
|---|---|---|---|---|---|
| Unit (mAbs.) | | | | | |

### 5. Observation

(1) In Comparative Example 3, in which no antibody was added, a sIL-2R concentration-dependent increase in absorbance was observed, whereas in Comparative Example 4 using the first reagent to which the 92204R-(WT) antibody was added, a sIL-2R concentration-dependent increase in absorbance was not observed. Thus, it was shown that the addition of the 92204R-(WT) antibody inhibited the specific reaction between the sIL-2R in the sample and the antibody-bound latex.
(2) In Example 3 using the first reagent to which the 92204R-(Δ105-107) modified antibody was added, a sIL-2R concentration-dependent increase in absorbance was observed as in Comparative Example 3, and the degree of increase was also at the same level. Thus, it was shown that the 92204R-(Δ105-107) modified antibody had no effect on the specific reaction between the sIL-2R and the antibody-bound latex.
(3) Also, in Example 4 using the first reagent to which the 92204R-(CDR3-A) modified antibody was added, a sIL-2R concentration-dependent increase in absorbance was observed as in Comparative Example 3 and Example 3, and the degree of increase was also at the same level. Thus, it was shown that the 92204R-(CDR3-A) modified antibody had no effect on the specific reaction between the sIL-2R and the antibody-bound latex.
(4) As can be seen from the above, by modifying the CDR amino acid sequence of the specific antibody, it was possible to obtain a non-specific reaction inhibitor that does not affect the specific reaction in an immunoassay method and acts as a decoy.

### [Test Example 4] Measurement of sIL-2R (LTIA method)

The concentration of sIL-2R in the sample was measured using a LTIA reagent to which the modified antibody of the present invention was added.

### 1. Measurement reagent

### [Comparative Example 5] (sIL-2R concentration measurement by CLEIA method)

Lumipulse Presto (registered trademark) IL-2R (Fujirebio Inc.) was used. The CLEIA method performs a B/F separation operation and has a washing step. Therefore, this method was used as a comparative example because it is a measurement method that is not easily affected by non-specific reactions originating from the sample.

### [Comparative Example 6] (sIL-2R concentration measurement by LTIA method)

A first reagent and a second reagent were prepared according to the method described in JP-A-2017-181377.

### [Example 5] (sIL-2R concentration measurement by LTIA method with addition of 92204R-(Δ105-107) modified antibody)

Reagents were prepared in the same manner as in Comparative Example 6, except that 92204R-(Δ105-107) modified antibody was added to the first reagent at a concentration of 100 µg/mL or 500 µg/mL.

### [Example 6] (sIL-2R concentration measurement by LTIA method with addition of 92204R-(CDR3-A) modified antibody)

Reagents were prepared in the same manner as in Comparative Example 6, except that 92204R-(CDR3-A) modified antibody was added to the first reagent at a concentration of 100 µg/mL or 500 µg/mL.

### 2. Sample

Samples used were samples (control samples: sample numbers 1 to 5) that show values close to the values measured by chemiluminescent enzyme immunoassay (CLEIA method) (Comparative Example 5), and samples (deviated samples: sample numbers 13 to 16) that exhibit a non-specific reaction and greatly deviate from the measured values of Comparative Example 5.

### 3. Measurement procedure

(1) In Comparative Example 5, measurement was performed using Lumipulse (registered trademark) -L2400 (Fujirebio Inc.) according to the package insert of the reagent.
(2) In Comparative Example 6, Example 5, and Example 6, the first reagents and the second reagents were combined, and the sIL-2R concentration in the sample was measured using a Hitachi 7180 automatic analyzer. Specifically, 120 µL of the first reagent was added to 5.6 µL of the sample and kept at 37 °C for 5 minutes, and then 40 µL of the second reagent was added and stirred. The absorbance change associated with agglutination formation was then measured over a period of 5 minutes at a main wavelength of 570 nm and a sub wavelength of 800 nm, and the measured value was calculated by applying the amount of absorbance change to a calibration curve obtained by measuring a standard substance with a known concentration.

### 4. Measurement result

The measurement results are shown in Table 3.

**[Table 3]**

| (U/mL) | | | | | | | |
|---|---|---|---|---|---|---|---|
| Type | Sample No.. | Comp.Ex.5 | Comp. Ex. 6 | Example 5 | | Example 6 | |
| | | CLEIA method | No antibody addition | 92204R-(Δ105-107) modified antibody | | 92204R-(CDR3-A) modified antibody | |
| | | | | 100µg/m L addition | 500µg/ml addition | 100µg/m L addition | 500µg/ml addition |
| Control sample | 1 | 278 | 270 | 294 | - | 331 | - |
| | 2 | 513 | 542 | 541 | 559 | 533 | 515 |
| | 3 | 718 | 790 | 786 | - | 798 | - |
| | 4 | 1242 | 1338 | 1335 | - | 1327 | - |
| | 5 | 1977 | 2084 | 2101 | - | 2108 | - |
| Deviated sample | 13 | 331 | 835 | 652 | 451 | 696 | 487 |
| | 14 | 176 | 407 | 252 | 211 | 215 | 225 |
| | 15 | 730 | 1350 | 942 | 791 | 947 | 810 |
| | 16 | 274 | 479 | 396 | 363 | 401 | 366 |

| | | | | | | | |
|---|---|---|---|---|---|---|---|
| -:not tested | | | | | | | |

### 5. Results and observation

(1) With respect to the control samples (sample numbers 1 to 5) in which the measured value of the CLEIA method (Comparative Example 5) and the measured value of the LTIA method without adding antibody (Comparative Example 6) were equivalent, the values measured by LTIA using the modified antibody of the present invention (Examples 5 and 6) were also equivalent to Comparative Examples 5 and 6. Thus, it was found that the addition of the modified antibody of the present invention does not affect the measured values of samples that do not exhibit non-specific reactions.
(2) For sample number 13, the measured value using the CLEIA method (Comparative Example 5) was 331 U/mL, whereas the measured value using the LTIA method without adding antibody (Comparative Example 6) was 835 U/mL, indicating the large gap between the measured values. On the other hand, the values measured by LTIA (Examples 5 and 6) in which 100 µg/mL of the modified antibody of the present invention was added were 652 or 696 U/mL, which showed a tendency close to Comparative Example 5. Further, the values measured by LTIA (Examples 5 and 6) in which 500 µg/mL of the modified antibody was added were 451 or 487 U/mL, which showed a tendency even closer to the measurement value of CLEIA (Comparative Example 5). Similar results were obtained for samples 14 to 16.
(3) As can be seen from the above, in the LTIA method, which is a homogeneous immunoassay method, by allowing the modified antibody of the present invention to be present in the reaction system, non-specific reactions originating from the sample could be suppressed.

### [Test Example 5] sIL-2R measurement by ELISA method

The sIL-2R concentration in the sample was measured using an ELISA reagent to which the modified antibody of the present invention was added.

### 1. Measuring method

### [Comparative Example 7] (sIL-2R concentration measurement by CLEIA method)

The same method as in Comparative Example 5 was carried out.

### [Comparative Example 8] (sIL-2R concentration measurement by ELISA method)

Anti-sIL-2R monoclonal antibody (clone number 92204R) was diluted to 10 µg/mL with 20 mM phosphate buffer (pH 7.2; hereinafter referred to as PBS) containing 150 mM sodium chloride, and 100 µL of the resulting solution was dispensed into each well of a 96-well microplate. The resulting mixture was allowed to stand at room temperature for 1 hour.

After washing each well three times with 400 µL of PBS (hereinafter referred to as PBST) containing 0.05% Tween (registered trademark) 20, 200 µL of PBST containing 1% bovine serum albumin (hereinafter referred to as BSA-PBST) was added to perform blocking for 1 hour at room temperature. The resulting was used as an ELISA plate.

After washing each well of the ELISA plate three times with 400 µL of PBST, 100 µL of the sample diluted 20 times with a sample diluent (BSA-PBST with HBR-1 (SCANTIBODIES LABORATORY, INC.) added to 100 µg/mL) to each well, and the resulting was allowed to stand at room temperature for 1 hour. After washing each well with 400 µL of PBST three times, 100 µL of biotin-labeled anti-sIL-2R monoclonal antibody (clone number 92212) diluted to 1.0 µg/mL with BSA-PBST was dispensed into each well, and the mixture was allowed to stand at room temperature for 1 hour. After washing each well with 400 µL of PBST three times, 100 µL of HRP-labeled streptavidin (Thermo Fisher Scientific) diluted to 0.2 µg/mL with BSA-PBST was dispensed into each well, and the resulting mixture was allowed to stand at room temperature for 30 minutes. After washing each well three times with 400 µL of PBST, 50 µL of citric acid buffer (pH 5.0) containing 0.2% orthophenylenediamine and 0.02% hydrogen peroxide was added, and the resulting was allowed to stand at room temperature for 10 minutes. Then, the enzyme reaction was stopped by adding 100 µL of 4.5N sulfuric acid, and the absorbance at a wavelength of 492 nm was measured.

Using a sample with known concentration as a calibrator, the sIL-2R value in the sample was calculated.

### [Example 7] sIL-2R concentration measurement by ELISA method with addition of 92204R-(Δ105-107) modified antibody

Measurement was carried out in the same manner as in Comparative Example 8, except that the 92204R-(Δ105-107) modified antibody was added to the sample dilution solution shown in Comparative Example 8 at a concentration of 100 µg/mL.

### [Example 8] (sIL-2R concentration measurement by ELISA method with addition of 92204R-(CDR3-A) modified antibody)

Measurement was carried out in the same manner as in Comparative Example 8, except that the 92204R-(CDR3-A) modified antibody was added to the sample diluent shown in Comparative Example 8 at a concentration of 100 µg/mL.

### 2. Sample

Samples used were samples (control samples: sample numbers 17 to 19) that show values close to the values measured by chemiluminescent enzyme immunoassay (CLEIA method) (Comparative Example 7), and a sample (deviated sample: sample number 20) that exhibits a non-specific reaction and greatly deviates from the measured values of Comparative Example 7.

### 3. Measurement result

The measurement results are shown in Table 4.

**[Table 4]**

| (U/mL) | | | | | |
|---|---|---|---|---|---|
| Type | Sample No.. | Comp.Ex.7 | Comp.Ex.8 | Example 7 | Example 8 |
| | | CLEIA method | No antibody addition | 92204R-(Δ105-107) modified antibody | 92204R-(CDR3-A) modified antibody |
| Control sample | 17 | 513 | 458 | 354 | 449 |
| | 18 | 1242 | 1047 | 911 | 907 |
| | 19 | 1977 | 1848 | 1689 | 1744 |
| Deviated sample | 20 | 353 | 756 | 484 | 279 |

### 4. Results and observation

(1) With respect to the control samples (sample numbers 17 to 19) in which the measured value of the CLEIA method (Comparative Example 7) and the measured value of the ELISA method without adding antibody (Comparative Example 8) were equivalent, the values measured by ELISA using the modified antibody of the present invention (Examples 7 and 8) were also equivalent to Comparative Examples 7 and 8. Thus, it was found that the addition of the modified antibody of the present invention does not significantly affect the measured values of samples that do not exhibit non-specific reactions.
(2) For sample number 20, the measured value using the CLEIA method (Comparative Example 7) was 353 U/mL, whereas the measured value using the ELISA method without adding antibody (Comparative Example 8) was 756 U/mL, indicating the large gap between the measured values. On the other hand, the values measured by ELISA (Examples 7 and 8) in which 100 µg/mL of the modified antibody of the present invention was added were 484 or 279 U/mL, which both showed a tendency close to Comparative Example 7.
(3) As can be seen from the above, in the ELISA method, which is a heterogeneous immunoassay method, by allowing the modified antibody of the present invention to be present in the reaction system, non-specific reactions originating from the sample could be suppressed. It was also found that the method of suppressing non-specific reactions using this modified antibody is effective not only in the homogeneous measurement systems of Examples 1 to 6, but also in the heterogeneous measurement systems of Examples 7 and 8.

### [Test Example 6] Verification of non-specific agglutination suppressing effect of antibody-bound latex by adding CDR-modified antibodies (LTIA method 2)

A test described below was implemented to evaluate the degree of agglutination between components in a sample and one type of antibody-bound latex using a buffer solution to which the CDR-modified antibody of the present invention and an unmodified antibody were added.

Since the antigen brain natriuretic peptide (BNP) is not a multivalent antigen with multiple identical epitopes in its molecule, one type of antibody-bound latex alone cannot generally cause agglutinations even if the BNP in the sample and the antibody-bound latex react, so that the absorbance does not change. Therefore, the absorbance change that occurs in the evaluation method shown in this test example can be considered to be due to nonspecific agglutination of the nonspecific factor and the antibody-bound latex in the sample.

### 1. Production of CDR-modified anti-BNP monoclonal antibody

Based on the anti-BNP IgG monoclonal antibody 33236 produced by the hybridoma (NITE ABP-03799), three types of L chain CDR modified antibodies, 33236-(W51A) modified antibody, 33236-(K95A) modified antibody and 33236-(CDR3-A) modified antibody were produced (FIG. 16), following to the same method as in Test Example 1 above. All of the antibodies had significantly reduced reactivity to the antigen BNP.

### 2.1. Measurement reagent

### (1) Preparation of first reagent

[Comparative Example 9] The first reagent shown below was prepared.
   100mM Bicine buffer (pH 8.0)
   200mM KCl
   0.5% BSA
[Comparative Example 10] 33236-(WT) antibody (unmodified antibody) was added to the first reagent of Comparative Example 9 at a concentration of 100 µg/mL.
[Example 9] 33236-(W51A) modified antibody was added to the first reagent of Comparative Example 9 at a concentration of 100 µg/mL.
[Example 10] 33236-(K95A) modified antibody was added to the first reagent of Comparative Example 9 at a concentration of 100 µg/mL.
[Example 11] 33236-(CDR3-A) modified antibody was added to the first reagent of Comparative Example 9 at a concentration of 100 µg/mL.

### (2) Preparation of fragment antibody

Fragment antibody 33236F(ab')₂ was prepared from anti-BNP IgG monoclonal antibody 33236 produced by the hybridoma (NITE ABP-03799) according to a conventional method.

### (3) Preparation of second reagent containing one type of antibody-bound latex

33236F(ab')₂ antibody-bound latex was prepared as follows. An equal amount of 0.6 mg/mL 33236F(ab')₂ antibody solution was added to a 1% polystyrene latex solution (manufactured by Sekisui Medical Co., Ltd.) with an average particle size of 270 nm and the resulting was stirred at 4 °C for 1 hour. Then, an equal amount of 2% BSA solution was added and the resulting was stirred at 4°C for 1 hour to prepare a 33236F(ab') ₂ antibody-bound latex solution. The solution was adjusted with 5mM MOPS buffer (pH 7.0) to give an absorbance 5.25Abs. /mL at a wavelength of 600nm and used as the second reagent.

### 3. Sample

A sample (non-specific agglutination sample: sample 21) that exhibits a non-specific reaction and changes in absorbance only with one type of antibody-bound latex (33236F(ab')₂ antibody-bound latex) was used.

### 4. Measurement procedure

The first reagent and the second reagent were combined, and the degree of nonspecific agglutination between each sample and the 33236F(ab')₂ antibody-bound latex alone was evaluated using a Hitachi 7180 automatic analyzer. Specifically, 150 µL of the first reagent was added to 12 µL of the sample and kept at 37 °C for 5 minutes, followed by addition of 50 µL of the second reagent and stirring. The absorbance change associated with agglutination formation was then measured over a period of 5 minutes at a main wavelength of 570 nm and a sub wavelength of 800 nm.

### 5. Measurement result

The measurement results are shown in Table 5.

**[Table 5]**

| | | Agglutination degree (Change in absorbance) (mAbs.) | | | | |
|---|---|---|---|---|---|---|
| Type | Sample No. | Comp.Ex.9 | Comp.Ex. 10 33236-(WT) | Example 9 33236-(W51A) | Example 10 33236-(K95A) | Example 11 33236-(CDR3-A) |
| | | No antibody addition | antibody added | antibody added | antibody added | antibody added |
| Non-specific agglutination sample | 21 | 190.4 | -7.1 | -7.0 | -7.1 | -6.9 |

### 6. Observation

The effects of the present invention were studied from the results of Comparative Examples 9 to 10 and Examples 9 and 11.
(1) In Comparative Example 9 in which no antibody was added, the change in absorbance of the 33236F(ab')₂ antibody-bound latex alone was 2.0 mAbs. or more, and nonspecific agglutination was observed.
(2) In Comparative Example 10 using the first reagent having 33236-(WT) added thereto, the change in absorbance of the 33236F(ab')₂ antibody-bound latex was significantly reduced (2.0 mAbs. or less), and non-specific agglutination was suppressed. This indicates that the non-specific factors contained in the sample acted as a decoy by reacting with the 33236-(WT) antibody added to the first reagent, and suppressed the non-specific agglutination of the non-specific factors and the 33236F(ab') ₂ antibody-bound latex after the addition of the second reagent.
(3) In Example 9 using the first reagent having 33236-(W51A) modified antibody added thereto, as in Comparative Example 10, the change in absorbance of 33236F(ab')₂ antibody-bound latex was significantly reduced (2.0 mAbs. or less), and non-specific agglutination was suppressed.
(3) In Example 10 using the first reagent having 33236-(K95A) modified antibody added thereto, as in Comparative Example 10, the change in absorbance of 33236F(ab')₂ antibody-bound latex was significantly reduced (2.0 mAbs. or less), and non-specific agglutination was suppressed.
(3) In Example 11 using the first reagent having 33236-(CDR3-A) modified antibody added thereto, as in Comparative Example 10, the change in absorbance of 33236F(ab')₂ antibody-bound latex was significantly reduced (2.0 mAbs. or less), and non-specific agglutination was suppressed.
(6) From the above, it was found that, even if the measurement target is different from that in Test Example 2, the CDR-modified antibody of the present invention, when allowed to be present in the antigen-antibody reaction system, acts as a decoy and can thereby suppress non-specific agglutination of the antibody-sensitized latex caused by non-specific factors in the sample.

### [Test Example 7] Verification of the effect of addition of CDR modified antibody on specific reaction (LTIA method 2)

The concentration of BNP in the sample was measured using a LTIA reagent to which the modified antibody of the present invention was added.

### 1. Measurement reagent

(1) First reagent
   [Comparative Example 11] The first reagent shown in Comparative Example 9 was used.
   [Comparative Example 12] The first reagent shown in Comparative Example 10 was used.
   [Example 12] The first reagent shown in Example 9 was used.
   [Example 13] The first reagent shown in Example 10 was used.
   [Example 14] The first reagent shown in Example 11 was used.
(2) Second reagent

The second reagent of Nanopia (registered trademark) BNP-A (Sekisui Medical Co., Ltd.) was used.

### 2. Sample

The following samples were subject to measurement as samples with known concentrations.
(1) Nanopia BNP, Calibrator for BNP (Sekisui Medical Co., Ltd.)

### 3. Measurement procedure

The first reagent and the second reagent were combined, and the sample with known concentration (Nanopia BNP, Calibrator for BNP) was measured using a Hitachi 7180 automatic analyzer. Specifically, 150 µL of the first reagent was added to 12 µL of the sample and kept at 37 °C for 5 minutes, followed by addition of 50 µL of the second reagent and stirring. The absorbance change associated with agglutination formation was then measured over a period of 5 minutes at a main wavelength of 570 nm and a sub wavelength of 800 nm.

### 4. Measurement result

The measurement results are shown in Table 6.

**[Table 6]**

| | | Rl added antibody(100µg/mL) | | | | |
|---|---|---|---|---|---|---|
| Indicated BNP concentration | | Comp.Ex.11 | Comp. Ex. 12 33236-(WT) | Example 12 33236-(W51A) | Example 13 33236-(K95A) | Example 14 33236-(CDR3-A) |
| | | No antibody added | antibody added | antibody added | antibody added | antibody added |
| Cal① | 0.0 pg/ml | 6 | -1 | 1 | -4 | 0 |
| Cal② | 102.8 pg/ml | 60 | 10 | 50 | 46 | 41 |
| Cal③ | 214.4 pg/ml | 131 | 25 | 120 | 117 | 104 |
| Cal④ | 402.2 pg/ml | 256 | 52 | 234 | 229 | 223 |
| Cal⑤ | 921.7 pg/ml | 443 | 141 | 430 | 428 | 425 |
| Cal⑥ | 2058.0 pg/ml | 590 | 297 | 577 | 580 | 582 |
| | Influence on specific reaction | | Inhibition confirmed | No influence | No influence | No influence |

### 5. Observation

(1) In contrast to the BNP concentration-dependent absorbance measurement observed in Comparative Example 11 in which no antibody was added, Comparative Example 12 using the first reagent to which the 33236-(WT) antibody was added showed clear decrease in the absorbance. Thus, it was shown that the addition of the 33236-(WT) antibody inhibited the specific reaction between the BNP in the sample and the antibody-bound latex.
(2) In Example 12 using the first reagent to which the 33236-(W51A) modified antibody was added, a BNP concentration-dependent increase in absorbance was observed as in Comparative Example 11, and the degree of increase was also at the same level. Thus, it was shown that the 33236-(W51A) modified antibody had no effect on the specific reaction between the BNP and antibody-bound latex.
(3) In Example 13 using the first reagent to which the 33236-(K95A) modified antibody was added, a BNP concentration-dependent increase in absorbance was observed as in Comparative Example 11 and Example 12, and the degree of increase was also at the same level. Thus, it was shown that the 33236-(K95A) modified antibody had no effect on the specific reaction between the BNP and antibody-bound latex.
(4) Even in Example 14 using the first reagent to which the 33236-(CDR3-A) modified antibody was added, a BNP concentration-dependent increase in absorbance was observed as in Comparative Example 11 and Examples 12 and 13, and the degree of increase was also at the same level. Thus, it was shown that the 33236-(CDR3-A) modified antibody had no effect on the specific reaction between the BNP and antibody-bound latex.
(5) As can be seen from the above, even if the measurement target is different from Test Example 3, by modifying the CDR amino acid sequence of the specific antibody, it was possible to obtain a non-specific reaction inhibitor that does not affect the specific reaction in an immunoassay method and acts as a decoy.

### [Industrial Applicability]

According to the present invention, in a method for detecting a target antigen in a sample using a specific antibody that specifically binds to the antigen, a modified antibody of the specific antibody is used to perform the following steps:
a) a step of bringing the sample into contact with the modified antibody; and
b) a step of bringing the sample into contact with the specific antibody simultaneously with or after the step a),
which enables the suppression of non-specific reactions contained in the sample and the accurate measurement. Further, the modified antibody of the present invention exhibits the desired effect even at a low concentration ratio to the specific antibody, and can further reduce the influence on the antigen-antibody reaction. Therefore, the present invention can also provide a detection reagent containing the modified antibody and capable of suppressing non-specific reactions, and a non-specific reaction inhibitor containing the modified antibody as an active ingredient.

### Accession number

### [Reference to deposited biological material]

(1) Hybridoma producing antibody number 92204R
   (a) Name and address of the depositary with which the biological material was deposited: National Institute of Technology and Evaluation, Patent Microorganisms Depositary Room 122, 2-5-8 Kazusakamatari, Kisarazu, Chiba, Japan (zip/postal code 292-0818)
   (b) Date of deposit of biological material with the depository of (a)
      September 25, 2015
   (c) Accession number assigned to deposit by the depositary of (a): NITE BP-02123
(2) Hybridoma producing antibody number 33236
   (a) Name and address of the depositary with which the biological material was deposited: National Institute of Technology and Evaluation, Patent Microorganisms Depositary Room 122, 2-5-8 Kazusakamatari, Kisarazu, Chiba, Japan (zip/postal code 292-0818)
   (b) Date of deposit of biological material with the depository of (a)
      December 16, 2022
   (c) Accession number assigned to deposit by the depositary of (a): NITE BP-03799
      (Accession number: NITE ABP-03799)

## Claims

1. A detection method for detecting a target antigen in a sample using a specific antibody that specifically binds to the antigen, the method comprising:
a) a step of bringing the sample into contact with a modified antibody; and
b) a step of bringing the sample into contact with the specific antibody simultaneously with or after the step a);
wherein the modified antibody is an antibody in which part or all of variable region of an antibody light chain (hereinafter abbreviated as L chain) and/or an antibody heavy chain (hereinafter abbreviated as H chain) of the specific antibody is modified.

2. The detection method according to claim 1, wherein the modified antibody is modified at part or all of at least one complementarity determining region (hereinafter abbreviated as CDR) and/or its region related to a three-dimensional structure of the CDR out of six CDRs of the L chain and H chain of the specific antibody, and has at least one unmodified CDR which is different from said CDR.

3. The detection method according to claim 1 or 2, wherein a reactivity of the modified antibody to the target antigen is 30 % or less relative to a reactivity of the specific antibody to the target antigen.

4. The detection method according to any one of claims 1 to 3, wherein a concentration ratio of the modified antibody to the specific antibody in an antigen-antibody reaction system is 1 to 29 times.

5. The detection method according to any one of claims 1 to 4, wherein the modified antibody is an intact antibody or a fragment antibody.

6. The detection method according to any one of claims 1 to 5, wherein the specific antibody includes one or more antibodies, and the modified antibody is a modified product of at least one of the antibodies as the specific antibody.

7. The detection method according to any one of claims 1 to 6, wherein the specific antibody includes a first specific antibody and a second specific antibody, and the modified antibody includes a modified product of at least one of the first specific antibody and the second specific antibody.

8. The detection method according to any one of claims 1 to 7, wherein the specific antibody is an intact antibody or a fragment antibody containing a binding site for the target antigen.

9. The detection method according to any one of claims 1 to 8, which is a method based on a homogeneous method or a heterogeneous method.

10. The detection method according to any one of claims 1 to 9, wherein at least one of the antibodies as the specific antibody is an antibody bound to an insoluble carrier or labeled with a labeling substance.

11. The detection method according to claim 10, wherein the insoluble carrier is latex particles, a gold colloid, a magnetic particle, a well-shaped plate, or a porous membrane.

12. The detection method according to any one of claims 1 to 11, wherein the step of bringing the target antigen in the sample into contact with the specific antibody is carried out in a solution.

13. The detection method according to claim 12, further comprising a step of optically detecting a degree of aggregation of latex particles in the solution.

14. The detection method according to claim 13, wherein the step of optically detecting comprises a step of optically detecting color development of a coloring agent or amount of a labeling substance, wherein the coloring agent or the labeling substance reacts depending on an amount of antigen-antibody complex in the solution.

15. A detection reagent for detecting a target antigen in a sample using a specific antibody that specifically binds to the antigen, the detection reagent comprising:
(1) a specific antibody that specifically binds to the antigen; and
(2) a modified antibody in which part or all of variable region of a L chain and/or H chain of the specific antibody is modified.

16. The detection reagent according to claim 15, wherein the modified antibody is modified at part or all of at least one CDR and/or its region related to a three-dimensional structure of the CDR out of six CDRs of the L chain and H chain of the specific antibody, and has at least one unmodified CDR which is different from said CDR.

17. The detection reagent according to claim 15 or 16, wherein a reactivity of the modified antibody to the target antigen is 30 % or less relative to a reactivity of the specific antibody to the target antigen.

18. The detection reagent according to any one of claims 15 to 17, wherein a concentration ratio of the modified antibody to the specific antibody in an antigen-antibody reaction system is 1 to 29 times.

19. The detection reagent according to any one of claims 15 to 18, wherein the modified antibody is an intact antibody or a fragment antibody.

20. The detection reagent according to any one of claims 15 to 19, wherein the specific antibody includes one or more antibodies, and the modified antibody is a modified product of at least one of the antibodies as the specific antibody.

21. The detection reagent according to any one of claims 15 to 20, wherein the specific antibody includes a first specific antibody and a second specific antibody, and the modified antibody includes a modified product of at least one of the first specific antibody and the second specific antibody.

22. The detection reagent according to any one of claims 15 to 21, wherein the specific antibody is an intact antibody or a fragment antibody containing a binding site for the target antigen.

23. The detection reagent according to any one of claims 15 to 22, which is a reagent based on a homogeneous method or a heterogeneous method.

24. The detection reagent according to any one of claims 15 to 23, wherein at least one of the antibodies as the specific antibody is an antibody bound to an insoluble carrier or labeled with a labeling substance.

25. The detection reagent according to claim 24, wherein the insoluble carrier is latex particles, a gold colloid, a magnetic particle, a well-shaped plate, or a porous membrane.

26. A detection reagent kit for detecting a target antigen in a sample using a specific antibody that specifically binds to the antigen, the kit comprising reagents (1) and (2), wherein at least one of the reagents (1) and (2) comprise a modified antibody in which part or all of variable region of a L chain and/or H chain of the specific antibody is modified:
(1) a reagent containing a buffer solution, and
(2) a reagent containing the specific antibody.

27. A suppression method for suppressing non-specific reactions in a detection method for detecting a target antigen, the suppression method comprising using a combination of a specific antibody that specifically binds to a target antigen in a sample and a modified antibody in which part or all of variable region of a L chain and/or H chain of the specific antibody is modified.

28. A non-specific reaction inhibitor used together with a specific antibody that specifically binds to a target antigen in a sample, comprising an antibody in which part or all of variable region of a L chain and/or H chain of the specific antibody is modified.

29. An antibody used together with a specific antibody that specifically binds to a target antigen in a sample, which is an antibody in which part or all of variable region of a L chain and/or H chain of the specific antibody is modified.
